# EUROPEAN PATENT APPLICATION

(11) **EP 4 461 325 A1**
(43) Date of publication of application: **13.11.2024**
(21) Application number: 23737310.5
(22) Date of filing: 06.01.2023
(51) Int. Cl.: A61M 1/36

(54) **MEDICAL LIQUID DETECTION SHEET**

(30) Priority: 07.01.2022 JP 2022001710
(71) Applicant: Nipro Corporation, Settsu-shi, Osaka 566-8510 (JP)
(72) Inventor: YAMABE, Kohei, Osaka-shi, Osaka 531-8510 (JP); MATSUBAYASHI, Satsuki, Osaka-shi, Osaka 531-8510 (JP)
(74) Representative: Slingsby Partners LLP
(86) International application number: PCT/JP2023/000169
(87) International publication number: WO 2023/132362

(57) **Abstract**

A liquid detection sheet 10 for medical use including a base member 16 having electric insulation properties, and a first detection wiring 22 and a second detection wiring 24 provided remote from each other on the base member 16. The liquid detection sheet 10 is configured to detect leakage of a detection target liquid 6 such as blood based on a change in an electrical connection state between the first detection wiring 22 and the second detection wiring 24 due to sticking of the detection target liquid 6. A main detection region 37 in which the first detection wiring 22 and the second detection wiring 24 extend substantially parallel to each other is provided on the base member 16. A distance between the first detection wiring 22 and the second detection wiring 24 opposed to each other in the main detection region 37 is set within a range of 40 to 100 mm.

## Description

### TECHNICAL FIELD

This invention relates to a liquid detection sheet for medical use, which is used to detect blood leakage, etc. in the medical field.

### BACKGROUND ART

In the medical field, it is sometimes necessary to detect leakage of blood or other liquids at an early stage when they occur. For example, during dialysis or infusion, liquid leakage may occur when the needle slips out from the patient and blood or liquid medicine leaks from the puncture site, and it is necessary to detect and deal with the leak before it becomes a major problem. However, it is not practical for busy medical personnel to continuously monitor dialysis, infusion, and other procedures from start to finish, so the use of liquid detection devices that automatically detect liquid leakage is being considered.

Conventionally, for example, Japanese Unexamined Patent Publication No. JP-A-2012-196293 (Patent Document 1) has proposed a liquid detection device that detects liquid leakage based on a change in electrical resistance caused by a short circuit between two detection wirings due to blood or liquid medicine.

### BACKGROUND ART DOCUMENT

### PATENT DOCUMENT

Patent Document 1: JP-A-2012-196293

### SUMMARY OF THE INVENTION

### PROBLEM THE INVENTION ATTEMPTS TO SOLVE

However, with conventional liquid detection devices, before use, it is necessary to check that there are no disconnections in either of the two detection wirings that are routed in parallel. Since a relatively large check voltage is required, checking before use is troublesome.

Besides, perspiration, etc. of a patient may be mistakenly detected as the occurrence of liquid leakage, and there is a need to avoid false detection of liquids that do not need to be detected, such as perspiration, while effectively detecting detection target liquids such as blood and liquid medicine.

In addition, in Patent Document 1, since the sheet that constitutes the liquid detection region has a six-layer structure, the number of parts is large and the manufacturing is time-consuming.

Further, in conventional liquid detection devices, an external circuit is generally connected to the two detection wirings via a detachable connector, but there is a risk that the connector may not be properly connected electrically due to such factors as being connected at an angle. In addition, since the connector is often subjected to external force, there is a risk of misalignment of the connector or disconnection in the wiring of the connector. There was also a need to make it possible to easily attach the connector to the sheet that constitutes the liquid detection area.

It is an object of the present invention to provide a liquid detection sheet for medical use of novel structure which is able to solve at least one of the problems inherent in conventional liquid detection devices as described above.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

A first preferred embodiment provides a liquid detection sheet for medical use comprising: a base member having electric insulation properties; and a first detection wiring and a second detection wiring provided remote from each other on the base member, the liquid detection sheet being configured to detect leakage of a detection target liquid such as blood based on a change in an electrical connection state between the first detection wiring and the second detection wiring due to sticking of the detection target liquid, wherein a main detection region in which the first detection wiring and the second detection wiring extend substantially parallel to each other is provided on the base member, and a distance between the first detection wiring and the second detection wiring opposed to each other in the main detection region is set within a range of 40 to 100 mm.

According to the liquid detection sheet for medical use structured following the present preferred embodiment, the distance between the first detection wiring and the second detection wiring opposed to each other in the main detection region is set within the range of 40 to 100 mm. This makes it difficult to detect a relatively small amount of liquid, such as perspiration, while making it possible to effectively detect the detection target liquid that has accumulated to a certain amount on the base member. Therefore, the desired detection target liquid can be detected with high accuracy so as to detect, for example, highly dangerous blood leakage or the like with high reliability, and the frequency of occurrence of false detection caused by perspiration or the like can be reduced.

A second preferred embodiment provides a liquid detection sheet for medical use comprising: a base member having electric insulation properties; and a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein an upper surface of the base member on which the plurality of detection wirings are formed by printing is covered with nonwoven fabric, and the base member and the nonwoven fabric are adhered to each other by a heat-welding sheet.

According to the liquid detection sheet for medical use structured following the present preferred embodiment, the detection wirings are formed by printing on the base member, so that the liquid detection sheet has a three-layer structure with a small number of layers, thereby facilitating manufacturing, reducing costs, and the like. In addition, by providing nonwoven fabric covering the upper surface of the base member on which the detection wirings are formed by printing, a good tactile feeling can be provided to the portion of the liquid detection sheet which is to be in contact with the forearm of the patient or the like. Besides, the detection target liquid is absorbed by the nonwoven fabric and spreads out, thereby inhibiting the detection target liquid from spilling or sticking to the patient. In addition, by heating the heat-welding sheet with the base member, the heat-welding sheet, and the nonwoven fabric laminated, the base member and the nonwoven fabric can be adhered to each other without requiring an adhesive application process.

A third preferred embodiment provides a liquid detection sheet for medical use comprising: a base member having electric insulation properties; and a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein a liquid-impermeable member that prevents passage of the detection target liquid is arranged below the plurality of detection wirings.

According to the liquid detection sheet for medical use structured following the present preferred embodiment, it is difficult for the detection target liquid to pass through the liquid-impermeable member and leak downward, thereby detecting the detection target liquid that accumulates on the liquid-impermeable member with excellent reliability. In addition, by keeping the detection target liquid on the liquid-impermeable member, contamination of the bed due to sticking of the detection target liquid will be avoided, for example, when the liquid detection sheet is laid on the bed.

A fourth preferred embodiment provides the liquid detection sheet for medical use according to the third preferred embodiment, wherein terminals of the plurality of detection wirings are located at a widthwise center portion of the base member in an outer peripheral end of the base member.

The liquid detection sheet including the liquid-impermeable member cannot be used by being turned upside down. Thus, for example, if the terminals of the detection wirings, which are located at the outer peripheral end of the base member, are arranged biasedly in the width direction of the base member, the distance from the terminals of the detection wirings to the external device will change between the case where the liquid detection sheet is used by being laid below the left arm of the patient and the case where the liquid detection sheet is used by being laid below the right arm of the patient, thereby causing differences in the required length and handling of the external wiring connected to the terminals of the detection wirings. According to the liquid detection sheet structured following the present preferred embodiment, the terminals of the detection wirings are located at the widthwise center portion of the base member. Thus, even if it is not possible to use the liquid detection sheet by turning upside down, the difference in the handling of the external wiring is minimized between the case where the liquid detection sheet is used on the left side of the patient and the case where the liquid detection sheet is used on the right side of the patient, for example, and in both cases, the external wiring is less likely to become an obstacle.

A fifth preferred embodiment provides a liquid detection sheet for medical use comprising: a base member having electric insulation properties; and a first detection wiring and a second detection wiring provided remote from each other on the base member, the liquid detection sheet being configured to detect leakage of a detection target liquid such as blood based on a change in an electrical connection state between the first detection wiring and the second detection wiring due to sticking of the detection target liquid, wherein the second detection wiring is provided so as to surround the first detection wiring on the base member.

According to the liquid detection sheet for medical use structured following the present preferred embodiment, by connecting a check circuit to the two ends of the second detection wiring, the electrical state of the second detection wiring (disconnection, etc.) can be checked by applying a small check voltage. Besides, since the first detection wiring has a specific wiring mode with respect to the second detection wiring, the normal condition of the first detection wiring can be estimated by checking the second detection wiring.

That is, damage such as disconnection of the first detection wiring or the second detection wiring is generally caused by bending of the detection sheet. Here, in the present preferred embodiment, the first detection wiring and the second detection wiring, which detect the liquid by means of a change in the electrical connection state therebetween, are configured such that the second detection wiring surrounds the first detection wiring relatively nearby. Thus, bending of the first detection wiring is accompanied by bending of the second detection wiring, and disconnection due to bending of the first detection wiring alone is difficult to assume. Therefore, if the first detection wiring is disconnected due to bending of the base member, for example, the second detection wiring surrounding the first detection wiring will also be disconnected. Accordingly, the electrical state of the first detection wiring can be estimated by checking the electrical state of the second detection wiring.

In this way, the electrical abnormalities such as disconnection of the first detection wiring can be checked based on the electrical state of the second detection wiring, thereby making it easier to check the first and second detection wirings.

A sixth preferred embodiment provides the liquid detection sheet for medical use according to the fifth preferred embodiment, wherein the first detection wiring and the second detection wiring are each imparted with a comb teeth-like shape by opposite wiring parts extending substantially parallel to each other and a plurality of branched wiring parts extending from the respective opposite wiring parts in a direction of opposition of the opposite wiring parts, and the plurality of branched wiring parts of the first detection wiring and the plurality of branched wiring parts of the second detection wiring are arranged in a length direction of the opposite wiring parts so as to interdigitate with one another in the direction of opposition of the opposite wiring parts.

According to the liquid detection sheet for medical use structured following the present preferred embodiment, it is possible to prevent the spacing between the first detection wiring and the second detection wiring from becoming too large, thereby detecting the detection target liquid with high accuracy. Besides, the branched wiring parts interdigitate with one another in the direction of opposition of the opposite wiring parts, and the branched wiring parts are arranged in the length direction of the opposite wiring parts. This makes it possible to efficiently obtain a relatively large effective detection area.

A seventh preferred embodiment provides the liquid detection sheet for medical use according to the fifth or sixth preferred embodiment, wherein a first terminal is provided at one end portion of the first detection wiring, a pair of second terminals are provided at two end portions of the second detection wiring, and the first terminal and the pair of second terminals are arranged side by side in a straight line, and the first terminal is located between the pair of second terminals.

According to the liquid detection sheet for medical use structured following the present preferred embodiment, the first terminal provided at one end portion of the first detection wiring and the pair of second terminals provided at the two end portions of the second detection wiring are arranged side by side in a straight line, which facilitates connection of the detection circuit to the first terminal and the second terminals. In particular, the arrangement of the first terminal between the pair of second terminals makes it easier to arrange the second detection wiring so as to surround the first detection wiring.

An eighth preferred embodiment provides the liquid detection sheet for medical use according to any one of the fifth through seventh preferred embodiments, wherein the base member comprises a base sheet that is impermeable to the detection target liquid, the first detection wiring and the second detection wiring are formed by printing on an upper surface of the base sheet, and a liquid-permeable sheet that has electric insulation properties and allows passage of the detection target liquid is provided on the upper surface of the base sheet so as to cover the first detection wiring and the second detection wiring.

According to the liquid detection sheet for medical use structured following the present preferred embodiment, the detection target liquid accumulates on the base sheet because the base sheet is impermeable to the detection target liquid. Therefore, the amount of the detection target liquid on the base sheet gradually increases and the area of the detection target liquid on the base sheet becomes larger, which increases the possibility of the detection target liquid touching both the first detection wiring and the second detection wiring to be detected. Besides, the base sheet, which does not require liquid-permeable properties, can easily be made smooth, and by the first and second detection wirings being formed by printing on the base sheet, disconnection or the like of the first and second detection wirings due to printing defects is unlikely to occur.

The liquid-permeable sheet having liquid-permeable properties is overlapped on the base sheet, and the liquid-permeable sheet diffusely transmits the detection target liquid sticking from above. This facilitates conduction by the detection target liquid between the first and second detection wirings, thereby increasing the reliability of detection. Furthermore, since the flow of the detection target liquid on the base sheet is limited by the liquid-permeable sheet, the detection target liquid is unlikely to spill out of the base sheet and is easier to detect by accumulating on the base sheet, while avoiding contamination of the surrounding area by the detection target liquid. Additionally, the liquid-permeable sheet covering the first and second detection wirings formed by printing on the base sheet has electric insulation properties. Thus, even if the body of the patient comes into contact with the liquid-permeable sheet, for example, the body of the patient will not cause conduction between the first and second detection wirings, thereby preventing false detection.

A ninth preferred embodiment provides the liquid detection sheet for medical use according to any one of the fifth through eighth preferred embodiments, further comprising a state checking member connected to the two end portions of the second detection wiring and configured to check an electrical state of the second detection wiring by applying a check voltage to the second detection wiring.

According to the liquid detection sheet for medical use structured following the present preferred embodiment, the state checking member is connected to the two end portions of the second detection wiring, thereby checking the electrical state of the second detection wiring with a relatively small check voltage.

A tenth preferred embodiment provides a liquid detection sheet for medical use comprising: a base member having electric insulation properties; and a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein the plurality of detection wirings are connected to each other by a resistor at a position outside a main detection region configured to detect the leakage of the detection target liquid.

According to the liquid detection sheet for medical use structured following the present preferred embodiment, based on differences in energization resistance, checking for disconnection of the detection wirings and detection of the liquid can be performed by the same application mode of voltage.

Besides, for example, the resistor can be provided in the internal circuit of a connector connected to the liquid detection sheet, which provides a greater degree of freedom in selecting a resistor compared to the case where the resistor is provided in the main detection region of the liquid detection sheet. In addition, by arranging the resistor at a position outside the main detection region with which the detection target liquid comes into contact, the detection target liquid can be prevented from touching the resistor.

An eleventh preferred embodiment provides a liquid detection sheet for medical use comprising: a base member having electric insulation properties; and a first detection wiring and a second detection wiring provided remote from each other on the base member, the liquid detection sheet being configured to detect leakage of a detection target liquid such as blood based on a change in an electrical connection state between the first detection wiring and the second detection wiring due to sticking of the detection target liquid, wherein one of the first detection wiring and the second detection wiring includes a curved part spaced outward from an end of the other of the first detection wiring and the second detection wiring and extending in an arc-like shape.

According to the liquid detection sheet for medical use structured following the present preferred embodiment, in the length direction of one of the first and second detection wirings, the change in the remote distance between the aforementioned one detection wiring and the other of the first and second detection wirings is reduced, and the change in the amount of the detection target liquid that is detectable in the length direction of the one detection wiring can be minimized, thereby achieving highly reliable liquid detection.

A twelfth preferred embodiment provides a liquid detection sheet for medical use comprising: a base member having electric insulation properties; and a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein terminals of the plurality of detection wirings are arranged side by side in an outer peripheral portion of the base member, and positioning parts are provided to the base member on opposite sides in a direction of arrangement of the terminals and located close to the terminals, the positioning parts positioning a connector with respect to the base member, the connector including an external wiring electrically connected to the terminals.

According to the liquid detection sheet for medical use structured following the present preferred embodiment, the base member and the connector are positioned by the positioning parts at the locations close to the pair of terminals located at the two ends in the direction of arrangement of the terminals, so that the connector is properly positioned with respect to the pair of terminals at the two ends.

Besides, when there is an intermediate terminal between the pair of terminals at the two ends, the proper positioning of the connector with respect to the pair of terminals at the two ends also ensures that the connector is properly positioned with respect to the intermediate terminal located between the pair of terminals at the two ends. This makes it possible to stably connect the internal circuit of the connector with respect to all of the plurality of terminals.

Even if external force acts between the connector and the base member in the direction of pulling away from each other, unintended detachment of the connector and the base member is prevented, thereby maintaining the electrical connection state between the connector and the base member, and hence between the external wiring of the connector and the first and second detection wirings on the base member.

A thirteenth preferred embodiment provides the liquid detection sheet for medical use according to the twelfth preferred embodiment, wherein the base member comprises a base sheet that is impermeable to the detection target liquid, and the base sheet is provided with a positioning hole through which a positioning pin protruding from the connector is inserted, and the positioning parts include the positioning hole.

According to the liquid detection sheet for medical use structured following the present preferred embodiment, a proper connection between the plurality of terminals provided on the base sheet and the internal circuit of the connector is easily achieved by a simple structure in which the positioning pin on the connector side is inserted through the positioning hole of the base sheet. In particular, when the base member is a thin-walled base sheet, the positioning hole is easy to form and the positioning action is effectively achieved.

A fourteenth preferred embodiment provides the liquid detection sheet for medical use according to the thirteenth preferred embodiment, wherein an annular reinforcing part is provided at a periphery of an opening of the positioning hole of the base sheet, the annular reinforcing part protruding in a thickness direction of the base sheet.

According to the liquid detection sheet for medical use structured following the present preferred embodiment, when the positioning pin is pressed against the inner circumferential surface of the positioning hole by the action of external force, damage such as tearing of the base sheet is prevented by the reinforcing part. In addition, since the length of the positioning hole provided to the thin-walled base sheet is increased by the reinforcing part, the contact area between the positioning pin and the positioning hole is enlarged, which makes it possible to obtain the positioning action more advantageously and to expect improved durability due to dispersion of stress.

A fifteenth preferred embodiment provides a liquid detection sheet for medical use comprising: a base member having electric insulation properties; and a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein a connector including an internal circuit electrically connected to terminals of the plurality of detection wirings is provided, and an external wiring electrically connected to the internal circuit of the connector and extending to an outside from the connector is configured such that the external wiring extends from the internal circuit to a side on which the connector is connected to the terminals, and an extending end of the external wiring curves so as to make a U-turn and extends to the outside from the connector on a side opposite to the side on which the connector is connected to the terminals.

According to the liquid detection sheet for medical use structured following the present preferred embodiment, the external wiring extends from the internal circuit to the side on which the connector is connected to the terminals and the extending end of the external wiring curves so as to make a U-turn. Thus, for example, when the external wiring is pulled by the action of external force, damage to the external wiring itself or to the connected portion between the external wiring and the internal circuit or the like is prevented owing to deformation of the curved portion.

A sixteenth preferred embodiment provides a liquid detection sheet for medical use comprising: a base member having electric insulation properties; and a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein a connector including an internal circuit electrically connected to terminals of the plurality of detection wirings is provided, a distal end portion of the connector includes an upper jaw part and a lower jaw part that are relatively displaceable in a direction of mutual separation and in a direction of mutual approach, an external wiring is electrically connected to the terminals via the internal circuit of the connector by an outer peripheral portion of the base member being clasped between the upper jaw part and the lower jaw part in a vertical direction, and the lower jaw part of the connector arranged below the base member is configured such that an upper surface comprises a sloping surface that slopes downward toward a distal end of the connector attached to the base member whereby the lower jaw part becomes thinner in the vertical direction toward the distal end of the connector attached to the base member.

According to the liquid detection sheet for medical use structured following the present preferred embodiment, the lower jaw part becomes thinner in the vertical direction toward the distal end of the connector attached to the base member. This makes it easy to insert the lower jaw part of the connector into the under part of the base member placed on the bed, thereby facilitating connection operation of the connector to the base member placed on the bed.

A seventeenth preferred embodiment provides the liquid detection sheet for medical use according to the sixteenth preferred embodiment, wherein the connector includes an urging member that urges distal end portions of the upper jaw part and the lower jaw part attached to the base member in the direction of mutual approach.

According to the liquid detection sheet for medical use structured following the present preferred embodiment, the upper jaw part and the lower jaw part are urged in the direction of mutual approach by the urging member. Thus, the connector can be easily attached to the base member by clasping the base member between the upper and lower jaw parts by means of urging force of the urging member. Moreover, since the connector is maintained in the attached state to the base member by the urging force exerted on the upper and lower jaw parts by the urging member, no special locking operation or the like is required.

### EFFECT OF THE INVENTION

According to the present invention, the detection target liquid can be detected with high accuracy while suppressing false detection of, for example, perspiration of a patient or the like that does not require detection.

### BRIEF DESCRIPTION OF THE DRAWINGS

FIG. 1 is a plan view of a liquid detection device including a liquid detection sheet for medical use according to a first practical embodiment of the present invention.
FIG. 2 is a cross sectional view taken along line 2-2 of FIG. 1.
FIG. 3 is a cross sectional view of the liquid detection device shown in FIG. 1, corresponding to a cross section taken along line 3-3 of FIG. 1.
FIG. 4 is a view suitable for explaining a laminated structure of the liquid detection sheet shown in FIG. 1.
FIG. 5A is a view suitable for explaining the liquid detection device shown in FIG. 1 in use, illustrating the state in which no blood leakage is detected.
FIG. 5B is a view suitable for explaining the liquid detection device shown in FIG. 1 in use, illustrating the state in which blood leakage is detected.
FIG. 6 is a plan view of a liquid detection device including a liquid detection sheet for medical use according to a second practical embodiment of the present invention.
FIG. 7 is a perspective view of a connector constituting the liquid detection device shown in FIG. 6.
FIG. 8 is a plan view of the connector shown in FIG. 7.
FIG. 9 is a right side view of the connector shown in FIG. 7.
FIG. 10 is a rear view of the connector shown in FIG. 7.
FIG. 11 is a cross sectional view taken along line 11-11 of FIG. 10.
FIG. 12 is a cross sectional view taken along line 12-12 of FIG. 9.
FIG. 13 is a cross sectional view taken along line 13-13 of FIG. 9.
FIG. 14 is a view diagrammatically showing connection between a detection wiring and an internal circuit of the connector in the liquid detection device shown in FIG. 6.

### EMBODIMENTS FOR CARRYING OUT THE INVENTION

Hereinafter, practical embodiments of the present invention will be described with reference to the drawings.

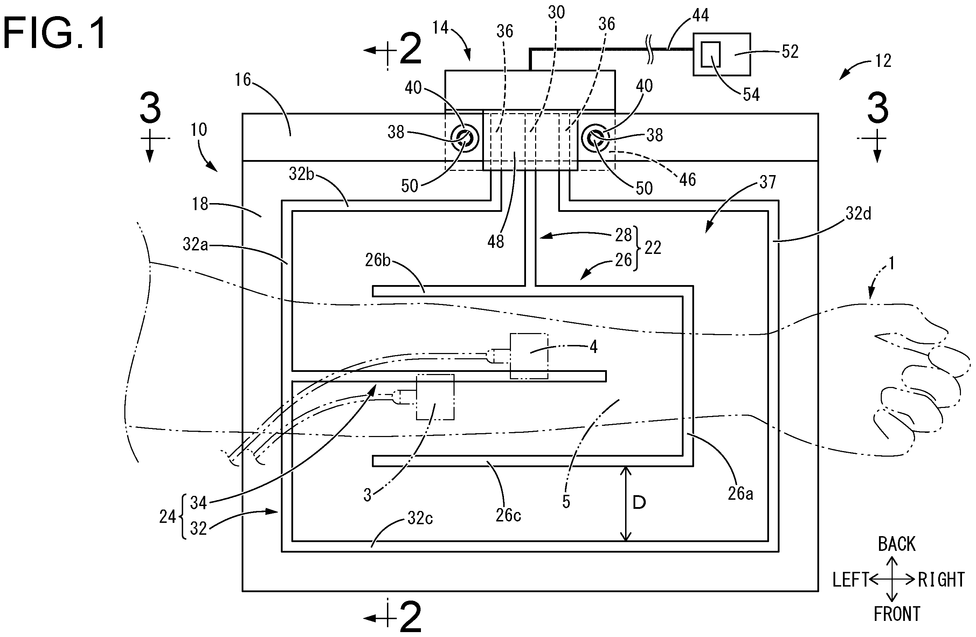

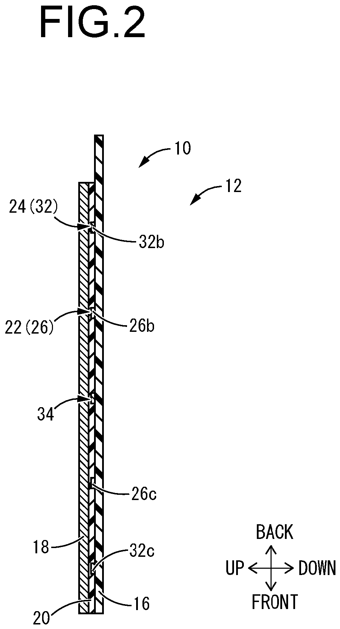

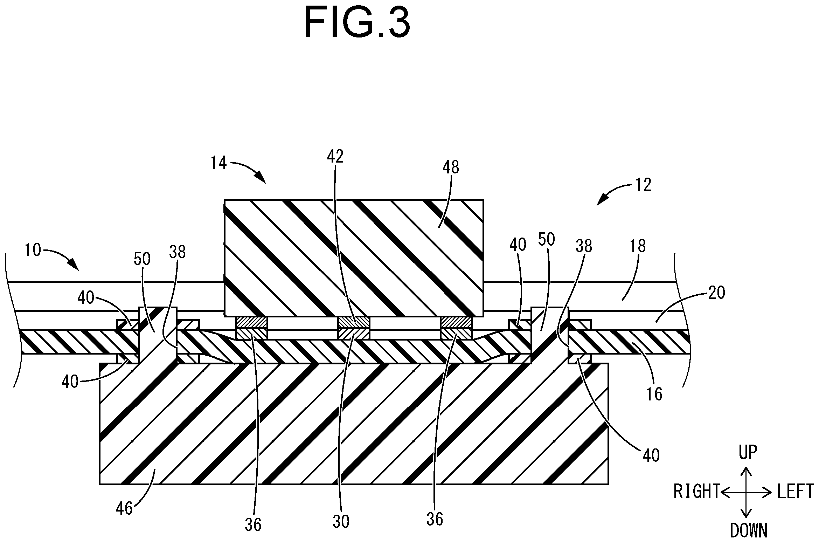

FIGS. 1 to 3 show a liquid detection device 12 including a liquid detection sheet 10 for medical use according to a first practical embodiment of the present invention. The liquid detection device 12 has a structure in which a connector 14 is attached to the liquid detection sheet 10. In the following description, as a general rule, the vertical direction refers to the vertical direction in FIG. 3, which is the vertical direction in the state of use, the front-back direction refers to the vertical direction in FIG. 1, and the left-right direction refers to the left-right direction in FIG. 1. In the drawings, the thickness of the components, the width of the detection wirings, and the like are exaggerated for illustrative purposes.

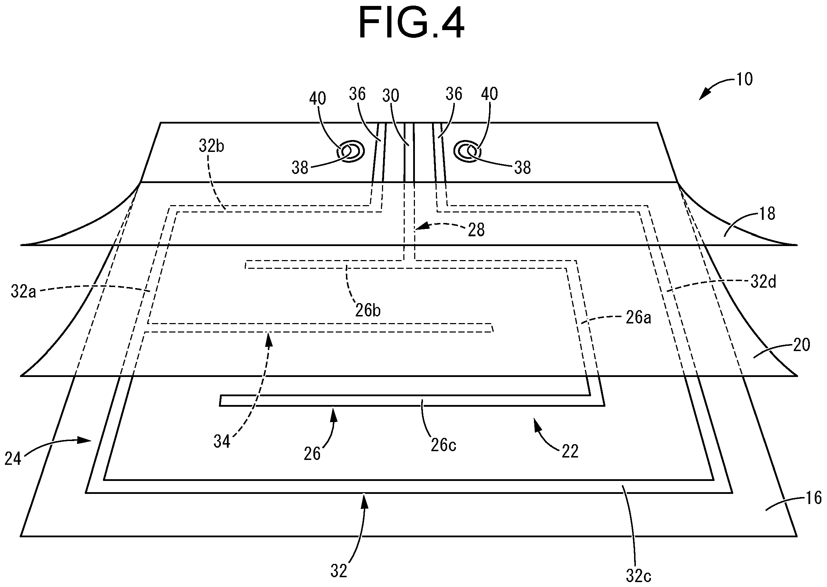

As shown in FIG. 4, the liquid detection sheet 10 has a laminated structure in which a base sheet 16 serving as a base member and a liquid-permeable sheet 18 overlapped on the upper surface of the base sheet 16 are fastened to each other by an adhesive sheet 20.

The base sheet 16 is in the form of a thin-walled sheet or film. The base sheet 16 is a liquid-impermeable member having liquid-impermeable properties that is not permeable to liquids such as blood. The base sheet 16 is made of an electrically insulating material. The base sheet 16 is made of a synthetic resin material, for example, and specifically, can be made of polyethylene terephthalate (PET). It is desirable that the thickness of the base sheet 16 be 0.01 mm to 0.5 mm from the viewpoint of, for example, minimizing sense of discomfort (stiffness) when a patient places his/her arm or the like on the liquid detection sheet 10 while ensuring the necessary electric insulation properties, and in this practical embodiment, the thickness is 0.05 mm, for example.

On the base sheet 16, a first detection wiring 22 and a second detection wiring 24 are provided. Both the first detection wiring 22 and the second detection wiring 24 are made of a conductive material, for example, a conductive paste made of a synthetic resin material mixed with a conductive filler made of a conductive material such as metal and carbon. The first detection wiring 22 and the second detection wiring 24 made of a conductive paste are each formed by printing in a predetermined shape (pattern) on the upper surface of the base sheet 16. Accordingly, the liquid-impermeable base sheet 16 is arranged below the first detection wiring 22 and the second detection wiring 24.

As shown in FIG. 1, the first detection wiring 22 includes a first central detection part 26 having a laterally rotated U shape with the left side open at the center portion of the base sheet 16, and also includes a terminal connecting part 28 extending straightly from the first central detection part 26 toward the back of the base sheet 16. At the back end of the terminal connecting part 28 in the first detection wiring 22, there is provided a first terminal 30 that is located at the back end of the base sheet 16 and is electrically connected to the connector 14 described below.

The second detection wiring 24 is arranged remote from the first detection wiring 22 and is provided so as to surround the first detection wiring 22. That is, the second detection wiring 24 includes an outer circumferential detection part 32 surrounding the first central detection part 26 of the first detection wiring 22, and also includes a second central detection part 34 extending from the outer circumferential detection part 32 so as to be inserted into the left side opening of the first central detection part 26 of the first detection wiring 22. The outer circumferential detection part 32 of the second detection wiring 24 is arranged such that its two end portions are disposed remote from each other on the left-right opposite sides of the terminal connecting part 28 of the first detection wiring 22 and are located at the back end of the base sheet 16. A pair of second terminals 36, 36 are provided at the two end portions of the outer circumferential detection part 32 of the second detection wiring 24. The pair of second terminals 36, 36 are disposed remote from each other on the left-right opposite sides of the first terminal 30, and those first terminal 30 and second terminals 36, 36 are arranged side by side in a straight line in the left-right direction. Thus, in this practical embodiment, the direction of arrangement of the first terminal 30 and the second terminals 36, 36 is the left-right direction. The first terminal 30 and the second terminals 36, 36 are suitably provided at the center portion of the base sheet 16 in the left-right width direction, and more suitably, they are provided approximately lineally symmetrically with respect to the left-right center line of the base sheet 16. This allows the connector 14 to be positioned at the center portion of the base sheet 16 in the left-right direction, with the connector 14 positioned on the opposite side (outside) of the body of a patient (1), regardless of whether the liquid detection sheet 10 is used on the right or left forearm (5) of the patient (1) in the state of use described below. Thus, for example, the distance from the connector 14 to a detector (52), which is described below, does not change significantly, making it easier to avoid excessive or insufficient wiring length.

A left end 32a of the outer circumferential detection part 32 in the second detection wiring 24 extends in the front-back direction, and is approximately parallel to a right end 26a of the first central detection part 26 in the first detection wiring 22, which extends in the front-back direction, so as to be opposite to each other in the left-right direction. The right end 26a extending in the front-back direction in the first central detection part 26 and the left end 32a extending in the front-back direction in the outer circumferential detection part 32 serve as opposite wiring parts of this practical embodiment. The first central detection part 26 includes front and back ends 26b, 26c extending leftward from the right end 26a. On the front-back opposite outer sides of the first central detection part 26, there are arranged front and back ends 32b, 32c of the outer circumferential detection part 32 extending rightward from the left end 32a of the outer circumferential detection part 32. Between the front and back ends 26b, 26c of the first central detection part 26, the second central detection part 34 extends rightward from the left end 32a of the outer circumferential detection part 32. In this way, the first detection wiring 22 and the second detection wiring 24 include branched wiring parts 26b, 26c, 32b, 32c, 34 extending straightly from the respective opposite wiring parts 26a, 32a in the direction of opposition of those opposite wiring parts 26a, 32a. Accordingly, the first detection wiring 22 and the second detection wiring 24 are each imparted with an approximately comb-teeth shape in the portions constituted by the opposite wiring parts 26a, 32a and the branched wiring parts 26b, 26c, 32b, 32c, 34. Thus, the branched wiring parts 26b, 26c of the first detection wiring 22 and the branched wiring parts 32b, 32c, 34 of the second detection wiring 24 are arranged alternately at approximately regular intervals in the front-back direction so as to interdigitate with one another in the left-right direction.

Therefore, the right end 26a in the first central detection part 26 of the first detection wiring 22 and the left end 32a in the outer circumferential detection part 32 of the second detection wiring 24 extend parallel to each other, while the front and back ends 26b, 26c in the first central detection part 26 and the front and back ends 32b, 32c and the second central detection part 34 of the second detection wiring 24 extend parallel to one another. Accordingly, those portions in the first and second detection wirings 22, 24 that extend parallel to and are opposite to one another constitute a main detection region 37 of this practical embodiment. The main detection region 37 of this practical embodiment is set to the radially inner portion of the liquid detection sheet 10 surrounded by the outer circumferential detection part 32 of the second detection wiring 24. In this practical embodiment, the right end 26a of the first central detection part 26 and the right end 32d of the outer circumferential detection part 32 are mutually remote and extend in parallel, and the remote distance therebetween is approximately the same as the remote distance D between the front and back ends 26b, 26c of the first central detection part 26 and the front and back ends 32b, 32c of the outer circumferential detection part 32 as well as the second central detection part 34. The right end 26a of the first central detection part 26 and the right end 32d of the outer circumferential detection part 32 also constitute the main detection region 37.

The first detection wiring 22 and the second detection wiring 24 are remote from each other on the base sheet 16, and the distance D between the first central detection part 26 of the first detection wiring 22 and the outer circumferential detection part 32 as well as the second central detection part 34 of the second detection wiring 24 opposed to each other (the distance of opposition in the main detection region 37) is set within the range of 40 mm to 100 mm. In this practical embodiment, the first central detection part 26 of the first detection wiring 22, and the outer circumferential detection part 32 as well as the second central detection part 34 of the second detection wiring 24 both have a configuration constituted by combination of a plurality of straight portions (the right end 26a, the front end 26b, and the back end 26c in the first detection wiring 22, and the left end 32a, the front end 32b, and the back end 32c, the right end 32d, and the second central detection part 34 in the second detection wiring 24). The distance D between the first central detection part 26 of the first detection wiring 22 and the outer circumferential detection part 32 as well as the second central detection part 34 of the second detection wiring 24 opposed to each other refers to the distance between the straight portions of the first central detection part 26, the outer circumferential detection part 32 and the second central detection part 34 that extend approximately parallel to one another. In this practical embodiment, the distance D between the straight portions of the first central detection part 26, the outer circumferential detection part 32 and the second central detection part 34 is approximately constant. However, the distance between the straight portions of the first central detection part 26, the outer circumferential detection part 32 and the second central detection part 34 may be different for each straight portion, in which case the minimum distance between the straight portions is 40 mm or more and the maximum distance between the straight portions is 100 mm or less.

The back end of the base sheet 16 is provided with a pair of positioning holes 38, 38 serving as a positioning part. The positioning holes 38 perforate the base sheet 16 in the thickness direction, and have, for example, a circular cross-section. The pair of positioning holes 38, 38 are arranged remote from each other in the left-right direction, and are located on the left-right opposite outer sides of the pair of second terminals 36, 36. The positioning hole 38 is arranged close to the second terminal 36 in the left-right direction. For example, the distance between the positioning hole 38 and the second terminal 36 arranged adjacently is 30 mm or less, more suitably 15 mm or less.

An annular reinforcing part 40 is provided at the periphery of the opening of each positioning hole 38. The reinforcing part 40 is provided at the periphery of the opening of the positioning hole 38 so as to protrude outward in the thickness direction of the base sheet 16. The reinforcing part 40 is formed of, for example, synthetic resin or metal, and the deformation rigidity of the base sheet 16 is increased at the portion where the reinforcing part 40 is provided. The reinforcing part 40 may be formed integrally with the base sheet 16 or may be formed as a separate member from the base sheet 16 and fastened to the base sheet 16 by welding or other means. In this practical embodiment, the reinforcing parts 40 are provided at the peripheries of the openings on both the upper and lower sides of the positioning holes 38, but may be provided only at the periphery of the opening on either the upper or lower side, for example.

The liquid-permeable sheet 18 has liquid-permeable properties to allow passage of a detection target liquid, such as blood, and is also has electric insulation properties. The liquid-permeable sheet 18 may be woven or knitted fabric, but is constituted by, for example, nonwoven fabric. As shown in FIG. 4, the liquid-permeable sheet 18 is overlapped on the upper surface of the base sheet 16, and covers the first detection wiring 22 and the second detection wiring 24 provided on the upper surface of the base sheet 16. The liquid-permeable sheet 18 has approximately the same dimensions in the left-right direction and shorter dimensions in the front-back direction compared to the base sheet 16, and the back end portion of the base sheet 16 protrudes backward from the liquid-permeable sheet 18 to be exposed. Thus, the first terminal 30 and the second terminals 36, 36 are not covered by the liquid-permeable sheet 18, but are exposed to the outside behind the liquid-permeable sheet 18. In this practical embodiment, the pair of positioning holes 38, 38 arranged on the left-right opposite outer sides of the second terminals 36, 36 are located behind the liquid-permeable sheet 18, and are formed only on the base sheet 16.

The base sheet 16 and the liquid-permeable sheet 18 are coupled to each other by the adhesive sheet 20 serving as an adhesive layer. The adhesive sheet 20 is, for example, a synthetic resin sheet with glue layers on both sides. One glue layer is adhered to the base sheet 16 and the other glue layer is adhered to the liquid-permeable sheet 18, thereby holding the base sheet 16 and the liquid-permeable sheet 18 in an overlapped state. The adhesive sheet 20 has liquid-permeable properties to allow passage of the detection target liquid. The adhesive sheet 20 may be, for example, a heat-welding sheet. The heat-welding sheet is made of thermoplastic resin such as polyimide, polyester, or polyurethane, for example, and becomes adhesive upon heating and solidifies upon cooling. Therefore, the base sheet 16 and the liquid-permeable sheet 18 can be adhered to each other by heating the heat-welding sheet and then cooling it, with the heat-welding sheet (the adhesive sheet 20) overlapped on the upper surface of the base sheet 16 and interposed between the base sheet 16 and the liquid-permeable sheet 18. The adhesive sheet 20 (the heat-welding sheet) is able to allow passage of the detection target liquid by, for example, being made in a mesh pattern, even if it is not made of a liquid-permeable material. The base sheet 16 and the liquid-permeable sheet 18 may be coupled to each other by an adhesive, for example, without using the adhesive sheet 20. The heat-welding sheet may be formed by thermosetting resin such as epoxy resin.

The connector 14 is attached to the liquid detection sheet 10 having such a structure. The connector 14 includes an internal circuit 42 (see FIG. 3) which is connected to the first detection wiring 22 and the second detection wiring 24 of the liquid detection sheet 10, and includes an external wiring 44 which is electrically connected to the internal circuit 42 and extends to the outside. The internal circuit 42 of the connector 14 is connected via the external wiring 44 to a detector 52 outside (described later).

The connector 14 is attached to the back end portion of the liquid detection sheet 10. That is, the connector 14 includes a lower jaw part 46 that is overlapped on the lower surface of the base sheet 16, and an upper jaw part 48 that is overlapped on the upper surface of the base sheet 16. The base sheet 16 is clasped between those lower jaw part 46 and upper jaw part 48.

With the base sheet 16 clasped between the lower jaw part 46 and the upper jaw part 48 of the connector 14, in the internal circuit 42, as shown in FIG. 3, the portion exposed on the lower surface of the upper jaw part 48 is overlapped on and in contact with the first terminal 30 and the second terminals 36, 36. This electrically connects the internal circuit 42 of the connector 14 to the first detection wiring 22 and the second detection wiring 24.

The lower jaw part 46 is provided with a pair of positioning pins 50, 50. The positioning pin 50 projects upward from the lower jaw part 46, and has a circular transverse cross section corresponding to the hole cross section of the positioning hole 38 in the base sheet 16. The positioning hole 38 of this practical embodiment has an approximately cylindrical shape which can be inserted through the positioning hole 38. The pair of positioning pins 50, 50 are provided remote from each other in the left-right direction. The remote distance between the pair of positioning pins 50, 50 in the left-right direction is larger than the left-right dimension of the portion of the upper jaw part 48 located between the pair of positioning pins 50, 50. Thus, the upper jaw part 48 is located between the pair of positioning pins 50, 50 when the connector 14 is attached to the base sheet 16.

In the connector 14, when the base sheet 16 is clasped between the lower jaw part 46 and the upper jaw part 48, the positioning pins 50, 50 are inserted through the positioning holes 38, 38 of the base sheet 16. Accordingly, the connector 14 is positioned with respect to the base sheet 16 in the vertical direction by clasping the base sheet 16 between the lower jaw part 46 and the upper jaw part 48. Furthermore, the connector 14 is positioned with respect to the base sheet 16 in the front-back direction and in the right-left direction by inserting the pair of positioning pins 50, 50 provided to the lower jaw part 46 through the positioning holes 38, 38 of the base sheet 16. By so doing, the internal circuit 42 of the connector 14 and the first and second terminals 30, 36, 36 provided on the base sheet 16 are positioned in a state of mutual contact, thereby achieving a stable connection state. A locking mechanism may be provided to the connector 14 to hold the lower jaw part 46 and the upper jaw part 48 in the state of clasping the base sheet 16 therebetween. This makes it possible to prevent the lower jaw part 46 and the upper jaw part 48 from opening accidentally and to prevent the positioning pins 50, 50 from slipping out of the positioning holes 38, 38.

The positioning holes 38, 38 of the base sheet 16 are provided close to the second terminals 36, 36 on their left-right opposite outer sides, and the positioning action of the positioning holes 38, 38 and the positioning pins 50, 50 efficiently contribute to the positioning of the second terminals 36, 36 and the internal circuit 42. The first terminal 30 is located between the second terminals 36, 36, and if the second terminals 36, 36 and the internal circuit 42 are positioned, the first terminal 30 and the internal circuit 42 will automatically be positioned as well. Thus, the first and second terminals 30, 36, 36 can all be positioned with respect to the internal circuit 42 by means of the positioning holes 38, 38 and the positioning pins 50, 50.

By the positioning pins 50, 50 of the connector 14 being inserted through the positioning holes 38, 38 of the base sheet 16, even if, for example, a backward force is exerted on the connector 14 to pull it away from the base sheet 16, the connector 14 will not easily detach from the base sheet 16. Thus, the connection between the connector 14 and the base sheet 16, and hence the connection between the internal circuit 42 and the first and second terminals 30, 36, 36 are stably maintained.

The peripheries of the openings on the upper and lower sides of each positioning hole 38 are each provided with an annular reinforcing part 40. Therefore, for example, when external forces act between the connector 14 and the base sheet 16 in the front-back and/or left-right directions, it is possible to prevent damage to the base sheet 16 caused by the positioning pins 50 being pressed strongly against the inner circumferential surface of the positioning hole 38. Since the reinforcing part 40 protrudes outward in the thickness direction of the base sheet 16, the length of the positioning hole 38 is increased by the length of the reinforcing part 40. Thus, the contact area between the positioning pin 50 and the positioning hole 38 is increased, making it possible to obtain the positioning action more advantageously as well as to expect improved durability due to dispersion of stress.

As shown in FIG. 1, the external wiring 44 of the connector 14 connected to the liquid detection sheet 10 is connected to a detector 52. The detector 52 applies a detection voltage between the first detection wiring 22 and the second detection wiring 24 via the connector 14, and detects the detection target liquid on the liquid detection sheet 10 based on, for example, a change in the electrical connection state between the first detection wiring 22 and the second detection wiring 24.

Specifically, when the detection target liquid sticks to the liquid detection sheet 10 from above, the detection target liquid is first absorbed by the liquid-permeable sheet 18 and passes through the liquid-permeable sheet 18 while diffusing. The detection target liquid that has passed through the liquid-permeable sheet 18 reaches the upper surface of the base sheet 16. Since the base sheet 16 does not allow the liquid to pass through, the detection target liquid spreads on the base sheet 16, and as the amount of the detection target liquid increases, the area of the detection target liquid on the base sheet 16 increases. When the detection target liquid on the base sheet 16 exceeds a predetermined amount, it spreads across the first detection wiring 22 and the second detection wiring 24, and the first detection wiring 22 and the second detection wiring 24 are mutually conducted by the detection target liquid. As a result, the electrical resistance between the first and second detection wirings 22, 24 changes, and the detector 52 detects the presence of the detection target liquid on the base sheet 16 based on the change in the electrical connection state between the first and second detection wirings 22, 24 by applying a detection voltage between the first and second detection wirings 22, 24.

The detector 52 also includes a state checking member 54 that checks the electrical state of the second detection wiring 24, such as disconnection. That is, since the second terminals 36, 36, which are two ends of the second detection wiring 24, are both connected to the detector 52, the electrical state of the second detection wiring 24, such as presence or absence of disconnection, can be checked by applying a relatively small check voltage between the second terminals 36, 36.

Since the first detection wiring 22 is surrounded by the second detection wiring 24, it can be presumed that there is no disconnection in the first detection wiring 22 if the checking function of the detector 52 confirms that there is no disconnection in the second detection wiring 24. That is, the disconnection of the first and second detection wirings 22, 24 is almost always caused by bending of the base sheet 16, unless there is defective formation during manufacturing. Here, bending of the base sheet 16 rarely results in bending of only the first detection wiring 22 located on the radially inner side, and in many cases it also involves bending of the second detection wiring 24 located on the radially outer side. Therefore, it is rare that only the first detection wiring 22 is disconnected and not the second detection wiring 24, and there is no practical problem in presuming that the first detection wiring 22 is not disconnected either by the fact that the second detection wiring 24 is not disconnected.

In this practical embodiment, the first terminal 30 provided to the first detection wiring 22 is located between the second terminals 36, 36 provided to the two ends of the second detection wiring 24, which makes it easy to arrange the second detection wiring 24 to surround the first detection wiring 22. Also, since the first terminal 30 and the second terminals 36, 36 are arranged side-by-side in the left-right direction at the back end of the base sheet 16, it is easy to connect the first and second terminals 30, 36, 36 to the internal circuit 42 of the connector 14.

The first and second detection wirings 22, 24 are formed by printing on the surface of the base sheet 16. The base sheet 16 is made of a liquid-impermeable PET film or the like, and the surface is easily made smooth. Thus, the first and second detection wirings 22, 24 can be formed with high precision, and defects such as disconnection are unlikely to occur. Disconnection due to defective formation of the first detection wiring 22 at the manufacturing stage can be checked using a known tester.

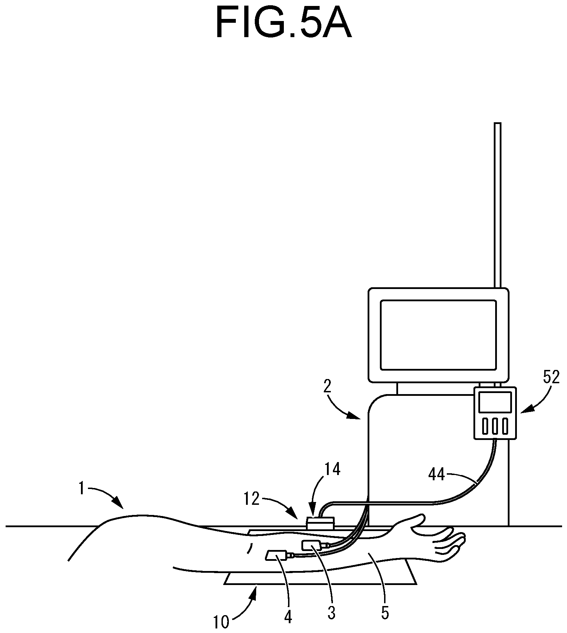

The liquid detection device 12 with such a structure is used as shown in FIG. 5A. That is, FIG. 5A shows a case in which a patient 1 uses the liquid detection device 12 during dialysis treatment using an artificial dialysis device 2, and the external wiring 44 extending from the connector 14 of the liquid detection device 12 is connected to the detector 52 attached to the artificial dialysis device 2. In FIG. 5A, a forearm 5 of the patient 1, which is punctured with a blood collection port 3 and a blood return port 4 of the artificial dialysis circuit, is placed on the liquid detection sheet 10 of the liquid detection device 12.

Although the forearm 5 of patient 1 is conductive, the liquid-permeable sheet 18 having electric insulation properties is interposed between the forearm 5 and the first and second detection wirings 22, 24. Thus, the change in electrical resistance value between the first and second detection wirings 22, 24 due to the forearm 5 is reduced, and false detection of liquid due to conductivity of the forearm 5 is prevented. Preferably, the part of the patient 1 that may leak blood or the like is positioned approximately in the center of the liquid detection sheet 10 and placed on the main detection region 37. In particular, in this practical embodiment, the forearm 5 of patient 1 is arranged so as to extend in the left-right direction, which is the direction of extension of the branched wiring parts 26b, 26c, 32b, 32c, 34 of the first and second detection wirings 22, 24. With this arrangement, there are fewer crossing points between the forearm 5 and the first and second detection wirings 22, 24 than when the forearm 5 is arranged so as to extend in the front-back direction. Thus, for example, false detection due to perspiration of the forearm 5 or the like is less likely to occur.

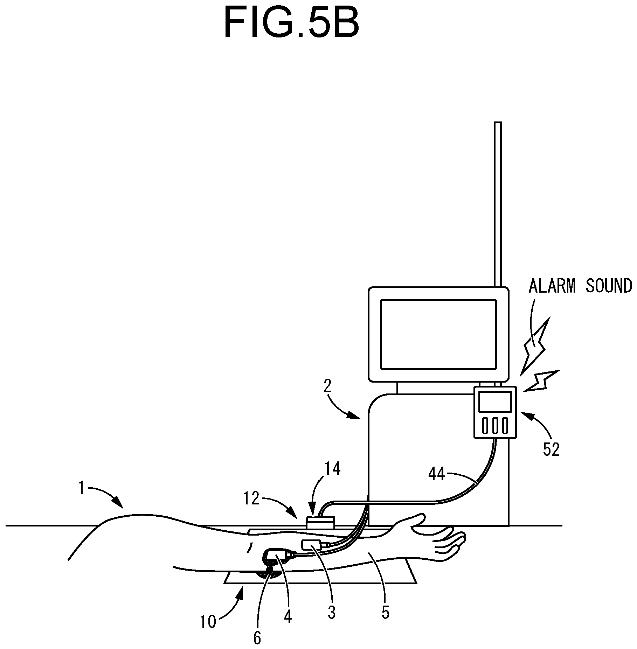

As shown in FIG. 5B, when blood 6 leaks out of the blood return port 4 due to, for example, slipping out of the needle, the blood 6 flows down onto the liquid detection sheet 10 laid under the forearm 5. The blood 6 that has flowed down is absorbed while being diffused by the liquid-permeable sheet 18 of the liquid detection sheet 10, passes through the liquid-permeable sheet 18, and reaches the base sheet 16. In the liquid detection sheet 10 of this practical embodiment, the blood 6 is absorbed by the liquid-permeable sheet 18 placed above the base sheet 16, thereby preventing, for example, the blood 6 from flowing over the base sheet 16 and spilling onto the bed or the floor.

When the amount of blood 6 reaching the base sheet 16 exceeds a predetermined amount of detected blood, the electrical insulation state between the first detection wiring 22 and the second detection wiring 24 changes due to the conductivity of the blood 6. Specifically, for example, by the blood spreading across the first detection wiring 22 and the second detection wiring 24 on the base sheet 16, the electrical resistance value between the first detection wiring 22 and the second detection wiring 24 decreases, and a current flows between the first detection wiring 22 and the second detection wiring 24 to which the detection voltage is applied. Based on the energization caused by the change in electrical resistance between the first detection wiring 22 and the second detection wiring 24, the detector 52 detects the presence of blood on the base sheet 16.

The diameter of the blood 6 that is absorbed by the liquid-permeable sheet 18 made of nonwoven fabric and spread on the base sheet 16 is approximately 40 mm for 0.5 mL and 100 mm for 2.5 mL. Generally, the blood flow rate in an artificial dialysis circuit is about 300 mL/min, and in this practical embodiment, the distance between the first detection wiring 22 and the second detection wiring 24 opposed to each other is within the range of 40 to 100 mm. Thus, blood leakage from the blood return port 4 can be detected quickly in a short time of about 0.1 to 0.5 seconds after the blood 6 reaches the liquid detection sheet 10.

The blood 6 absorbed by the liquid-permeable sheet 18 reaches the base sheet 16 while being diffused by the liquid-permeable sheet 18, thereby touching the first and second detection wirings 22, 24 more reliably and achieving detection of the blood 6 through conduction of the first and second detection wirings 22, 24. In particular, even when the forearm 5 is placed on the liquid detection sheet 10, by the blood 6 spreading within the liquid-permeable sheet 18, the spreading of the blood 6 is unlikely to be completely dammed up by the forearm 5, and stable detection by the spreading of the blood 6 can be expected. Also, the flow of the blood 6 on the base sheet 16 is limited by the liquid-permeable sheet 18, so that the blood 6 is prevented from spilling onto the bed, floor, etc., thereby preventing contamination or the like due to sticking of the blood 6.

The distance between the first detection wiring 22 and the second detection wiring 24 opposed to each other in the main detection region 37 is set within the range of 40 to 100 mm. Thus, relatively small amounts of liquid such as perspiration are unlikely to be detected, while the blood 6 that has accumulated to a predetermined amount on the base sheet 16 can be effectively detected. Therefore, the presence of the detection target liquid such as the blood 6, which is associated with high-risk events such as blood leakage during artificial dialysis, for example, can be detected with high reliability, and the frequency of occurrence of false detection caused by perspiration, etc., can be reduced.

Since the first detection wiring 22 and the second detection wiring 24, which are arranged remote from each other, are electrically insulated without being conducted through resistance or the like, a large electrical change, such as a decrease in electrical resistance value, can be obtained upon touch of the blood 6. Therefore, for example, false detection due to relatively small electrical changes based on factors other than touch of the blood 6 is less likely to occur, thereby improving accuracy and reliability of detection.

Besides, the first detection wiring 22 and the second detection wiring 24 are arranged in a comb-teeth shape in the main detection region 37, which extends parallel to each other, and the remote distance therebetween is approximately constant. This makes it possible to stably detect the blood 6 even if the position of the blood 6 accumulating on the base sheet 16 shifts slightly. Moreover, by arranging the branched wiring parts 26b, 26c of the first detection wiring 22 and the branched wiring parts 32b, 32c, 34 of the second detection wiring 24 at regular intervals such that they interdigitate with one another in the direction of opposition of the opposite wiring parts 26a, 32a, an effective detection area can be obtained efficiently and widely.

It is desirable that the detector 52 include a reporting device that reports the detection of liquid leakage to the outside. The reporting device is not limited in embodiment as long as it notifies the detection of the detection target liquid (the blood 6). For example, sounding an alarm sound, lighting an alarm light, or sending a notification to a mobile device or a nurse center are included. By providing such a reporting device, medical personnel and others can promptly recognize the occurrence of liquid leakage and take necessary treatments quickly. The reporting device of the detector 52 is not essential. For example, the detector 52 may send an emergency stop signal to the artificial dialysis device 2 upon detection of liquid leakage to automatically stop the artificial dialysis device 2, thereby preventing further leakage of the blood 6.

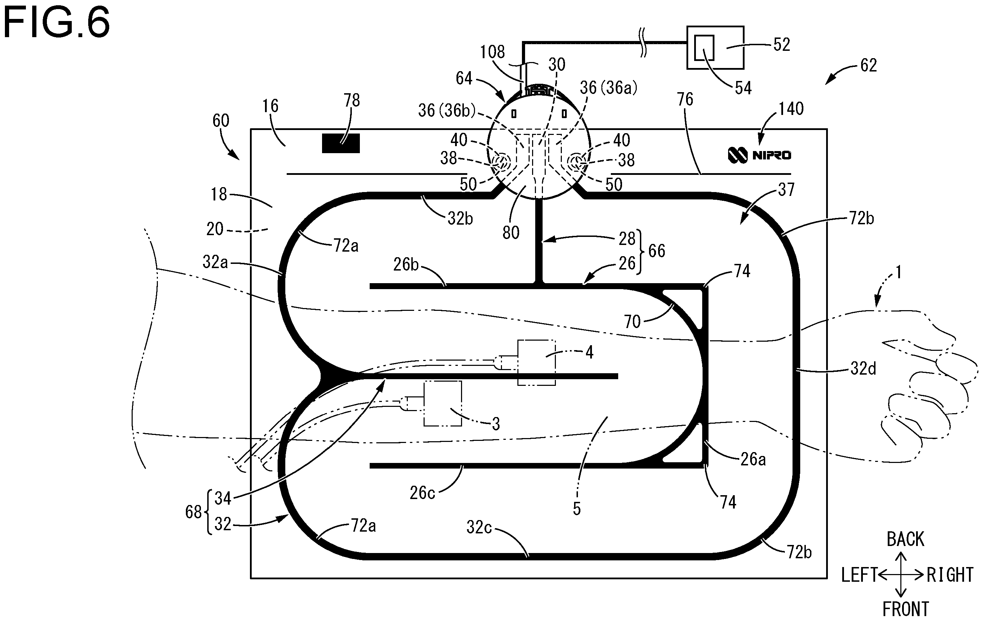

FIG. 6 shows a liquid detection device 62 for medical use including a liquid detection sheet 60 for medical use according to a second practical embodiment of the present invention. The liquid detection device 62 comprises the liquid detection sheet 60 including a connector 64. In the following description, components and parts that are substantially identical with those in the first practical embodiment will be assigned like symbols and not described in any detail.

Like the liquid detection sheet 10 of the first practical embodiment, the liquid detection sheet 60 has a three-layer structure in which the base sheet 16 serving as a base member and the liquid-permeable sheet 18 constituted by nonwoven fabric are fastened to each other in an overlapped state by the adhesive sheet 20 constituted by a heat-welding sheet.

On the upper surface of the base sheet 16, a first detection wiring 66 and a second detection wiring 68 are formed by printing. Like the first practical embodiment, the first and second detection wirings 66, 68 are formed by a conductive paste and are formed by printing in a predetermined shape on the upper surface of the base sheet 16. As in the first practical embodiment, the second detection wiring 68 is arranged remote from the first detection wiring 66 so as to surround it. Like the first practical embodiment, the liquid detection sheet 60 detects the presence of the detection target liquid on the base sheet 16 based on the change in the electrical connection state between the first and second detection wirings 66, 68. In FIG. 6, for ease of understanding, the liquid-permeable sheet 18 constituted by nonwoven fabric is made transparent to make the first and second detection wirings 66, 68 visible.

In the first and second detection wirings 66, 68 in this practical embodiment, change in the remote distance at the ends and corners is reduced. That is, the portion of the first detection wiring 66 arranged around the right end of the second central detection part 34 of the second detection wiring 68 constitutes a first curved part 70 serving as a curved part that curves and extends around the right end of the second central detection part 34. In this practical embodiment, the first curved part 70 has an approximately semi-circular arc shape, so that the distance between the first detection wiring 66 and the right end of the second central detection part 34 is approximately constant in the length direction of the first detection wiring 66.

Similarly, the portions of the second detection wiring 68 arranged around the left ends of the front and back ends 26b, 26c of the first detection wiring 66 constitute second curved parts 72a, 72a serving as curved parts that curve and extend around the left ends of the front and back ends 26b, 26c of the first detection wiring 66. In this practical embodiment, the second curved parts 72a, 72a each have an approximately semi-circular arc shape, so that the distance between the left ends of the front and back ends 26b, 26c and the second detection wiring 68 is approximately constant in the length direction of the second detection wiring 68.

The portion of the first detection wiring 66 that extends so as to surround the right end of the second central detection part 34 of the second detection wiring 68 has a double structure with corner-shaped parts 74, 74 provided on the radially outer side of the first curved part 70. The corner-shaped parts 74, 74 are the corners formed by the right end 26a and the front and back ends 26b, 26c, as in the first practical embodiment, and in this practical embodiment, they have an approximately right angle. The first curved part 70 and the corner-shaped parts 74, 74 may be integrated by filling the space therebetween with a conductive paste so as not to form a double structure. However, by providing a portion without the conductive paste between the first curved part 70 and the corner-shaped parts 74, 74 as in this practical embodiment, the amount of the conductive paste used can be reduced.

In the second detection wiring 68, the portions arranged on the radially outer side of the corner-shaped parts 74, 74 constitute a second curved parts 72b that curve and extend so as to surround the corner-shaped parts 74. The second curved part 72b is approximately arcuate with a central angle of 90° around the corner-shaped part 74, and the distance from the corner-shaped part 74 is approximately constant in the length direction.

As described above, the first curved part 70, the second curved parts 72a, 72a, 72b, 72b and the corner-shaped parts 74, 74 are provided. Accordingly, in the main detection region 37, the distance between the first central detection part 26 of the first detection wiring 66 and the second detection wiring 68 is approximately constant on both the radially inner and outer sides of the first central detection part 26.

Although the method of manufacturing the liquid detection sheet 60 having a three-layer structure is not particularly limited, the liquid detection sheet 60 is manufactured, for example, as follows.

First, a plurality of base sheets 16 are formed in a continuous band shape, and the first and second detection wirings 66, 68 are printed on each of those base sheets 16. Subsequently, the adhesive sheet 20 and the liquid-permeable sheet 18, also formed in a continuous band shape, are overlapped on the base sheet 16 in a continuous band shape, and the base sheet 16 and the liquid-permeable sheet 18 are heat-welded together by heating and then cooling the adhesive sheet 20. The liquid-permeable sheet 18 and the adhesive sheet 20 are adhered at a position that does not reach the back end of the base sheet 16 so that the first and second terminals 30, 36, 36 are exposed. Here, in this practical embodiment, a line 76 is printed on the base sheet 16 indicating the back end position of the liquid-permeable sheet 18 and the adhesive sheet 20. By checking the said line 76 with a camera, the relative position of the base sheet 16, the liquid-permeable sheet 18, and the adhesive sheet 20 in the front-back direction is defined.

Next, the band-shaped laminated sheet, in which the base sheet 16, the adhesive sheet 20, and the liquid-permeable sheet 18 are laminated, is cut in the length direction (the left-right direction of the liquid detection sheet 60 shown in FIG. 6), thereby completing the manufacture of the plurality of liquid detection sheets 60. In this practical embodiment, a marker 78 for checking the cutting position is printed on the upper surface of the back part of the base sheet 16 that is exposed without being covered by the liquid-permeable sheet 18. By checking the cutting position of the band-shaped laminated sheet by photographing the said marker 78 with a camera, for example, the band-shaped laminated sheet can be automatically cut into a plurality of liquid detection sheets.

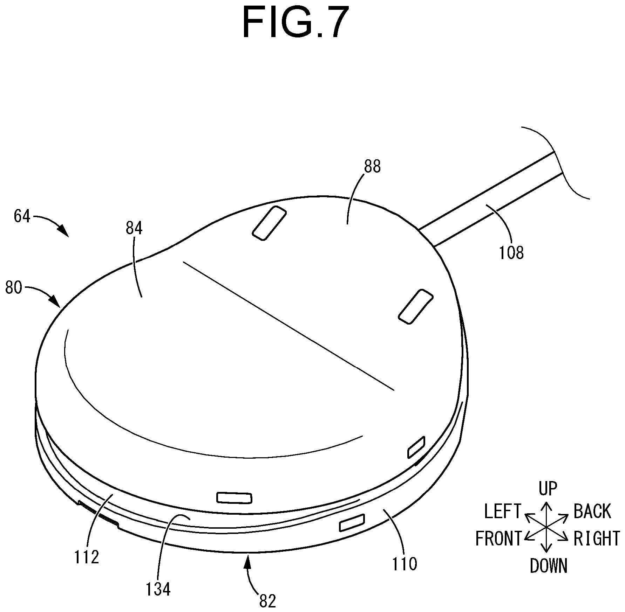

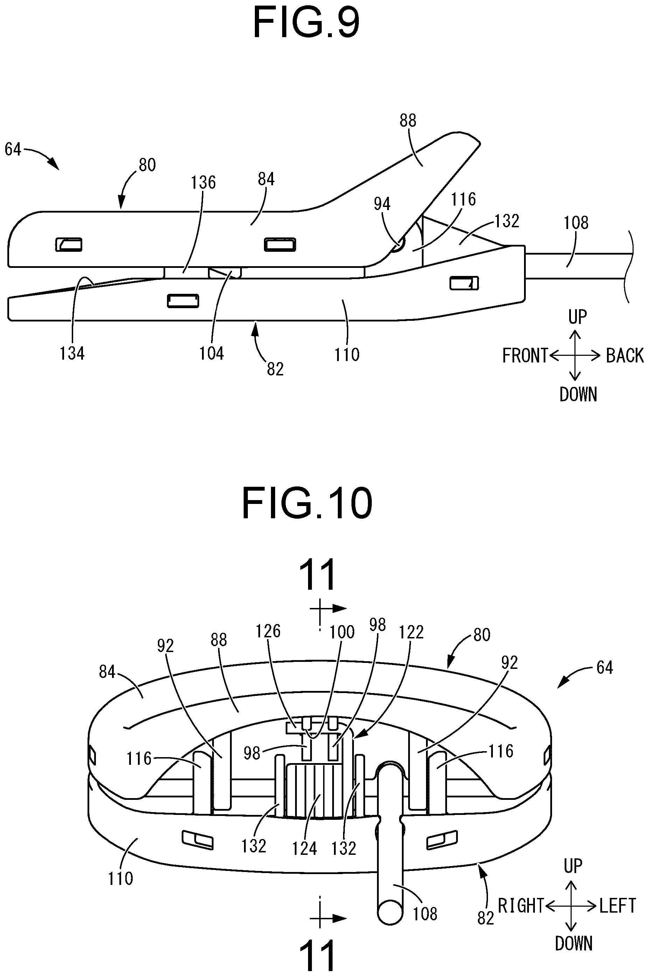

The connector 64 has a structure in which an upper jaw part 80 and a lower jaw part 82, each of which has a hollow structure, are coupled in a mutually tiltable manner, as shown in FIGS. 6 to 13.

The upper jaw part 80 has a hollow structure by combining an upper outer member 84 and an upper inner member 86. The upper outer member 84 has a shallow-bottomed cylindrical shape opening downward, and the end (the back part) opposite to the side of the connector 64 connected to the liquid detection sheet 60 (the base sheet 16) constitutes an operating part 88 that slopes upward toward the back. The upper inner member 86 has an approximately flat plate shape, and its back end does not reach the back end of the operating part 88. The upper outer member 84 and the upper inner member 86 are mechanically coupled to each other by, for example, a locking claw provided at the outer peripheral end of the upper inner member 86 being inserted and locked into a hole opening in the peripheral wall portion of the upper outer member 84. The upper outer member 84 and the upper inner member 86 may be fixed to each other by welding or adhesion, for example.

The upper outer member 84 and the upper inner member 86 are each provided with a plurality of reinforcing ribs 90 protruding from the respective inner surfaces opposed in the vertical direction. Thus, improvement in deformation rigidity can be achieved by the reinforcing ribs 90 while making the respective outer surfaces of the upper outer member 84 and the upper inner member 86 smooth in shape. The number, arrangement, cross-sectional shape, size, etc. of the reinforcing ribs 90 are set appropriately based on, for example, the deformation rigidity required of the upper jaw part 80 or the like.

A pair of shaft support parts 92, 92 projecting downward are integrally formed in the operating part 88 of the upper outer member 84. The shaft support parts 92 are in the form of plates that are opposed to each other in the left-right direction, and include a pair of spindle parts 94, 94 projecting outward in the left-right direction. The spindle parts 94 are approximately cylindrical in shape, and the lower portion of the projecting distal end portion is cut diagonally to provide a first guide surface 96 that slopes upward toward the projecting distal end side.

First support ribs 98 are provided between the opposed pair of shaft support parts 92. The first support ribs 98 project downward from the upper outer member 84, and in this practical embodiment, are in the form of a pair of plates that are opposed to each other in the left-right direction. The first support rib 98 is penetrated by a first notch 100, which opens downward, in the thickness direction (the left-right direction).

An internal substrate 102 is housed inside the upper jaw part 80. The internal substrate 102 has a structure in which the internal circuit 42 is mounted on an insulating substrate made of resin, for example, by means of printed wiring, etc. In this practical embodiment, the internal substrate 102 is a printed circuit board with electronic components attached to a printed wiring board to provide the internal circuit 42 serving as an electric circuit, and is securely supported by machine screws, positioning ribs, or the like to at least one of the upper outer member 84 and the upper inner member 86. The internal substrate 102 includes internal terminals 104, 104, 104 projecting downward. The internal terminals 104 are electrically connected to the internal circuit 42 on the internal substrate 102, and are exposed downward via through holes 106 that penetrates the upper inner member 86. By the connector 64 being attached to the base sheet 16, the internal terminals 104, 104, 104 are electrically connected to the first terminal 30 and the second terminals 36, 36 of the first and second detection wirings 66, 68, so that the internal circuit 42 is electrically connected to the first and second terminals 30, 36, 36 via the internal terminals 104, 104, 104. Whereas the configuration of the internal circuit 42 is not limited, the internal circuit 42 is equipped with, for example, a control circuit that applies a detection voltage to the first and second detection wirings 66, 68, a detection circuit that measures the current from the first and second detection wirings 66, 68 to detect disconnection and liquid leakage, a transmission circuit that transmits the detection results to the outside through an external wiring 108 described later or wirelessly, and the like.

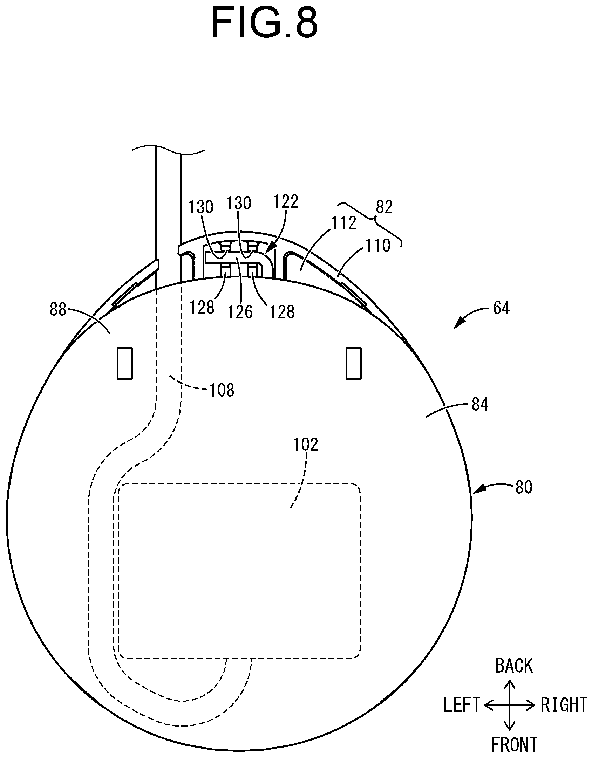

Inside the upper jaw part 80, the end of the external wiring 108 connected to the internal circuit 42 is housed. As shown in FIG. 8, the external wiring 108 extends forward from the edge on the front side (the lower side in FIG. 8) of the internal substrate 102 equipped with the internal circuit 42, which is the side connected to the liquid detection sheet 60, and the extending end is curved and routed inside the connector 64 so as to make a U-turn backward, with the U-turn curved end extends backward along the lateral side of the internal substrate 102, so as to extend backward to the outside from the connector 64 to be exposed. By so doing, even if the external wiring 108 exposed and extending out from the connector 64 is pulled backward, etc., the external wiring 108 is curved and extends inside the upper jaw part 80, thereby preventing disconnection of the external wiring 108, disconnection due to breakage of the connected portion between the external wiring 108 and the internal circuit 42, or the like owing to deformation of the curved portion. The external wiring 108 can be positioned appropriately inside the connector 64 by utilizing the reinforcing ribs 90 or the like, in order to suppress the transmission of tensile force, which is exerted from the outside, to the connected portion to the internal circuit 42.

The lower jaw part 82 has a hollow structure in which a lower outer member 110 and a lower inner member 112 are combined. Like the upper outer member 84 and the upper inner member 86 of the upper jaw part 80, the lower outer member 110 and the lower inner member 112 may be mechanically fixed or may be mutually fixed by means of welding, adhesion, or the like.

A plurality of reinforcing ribs 114 are provided on the respective opposed inner surfaces of the lower outer member 110 and the lower inner member 112. Thus, improvement in deformation rigidity can be achieved by the reinforcing ribs 114 while making the respective outer surfaces of the lower outer member 110 and the lower inner member 112 smooth in shape. The back end of the lower inner member 112 reaches the back end of the lower outer member 110 on the opposite portions in the left-right direction, and does not reach the back end of the lower outer member 110 at the center portion in the left-right direction where a spring member 122, described later, is arranged.

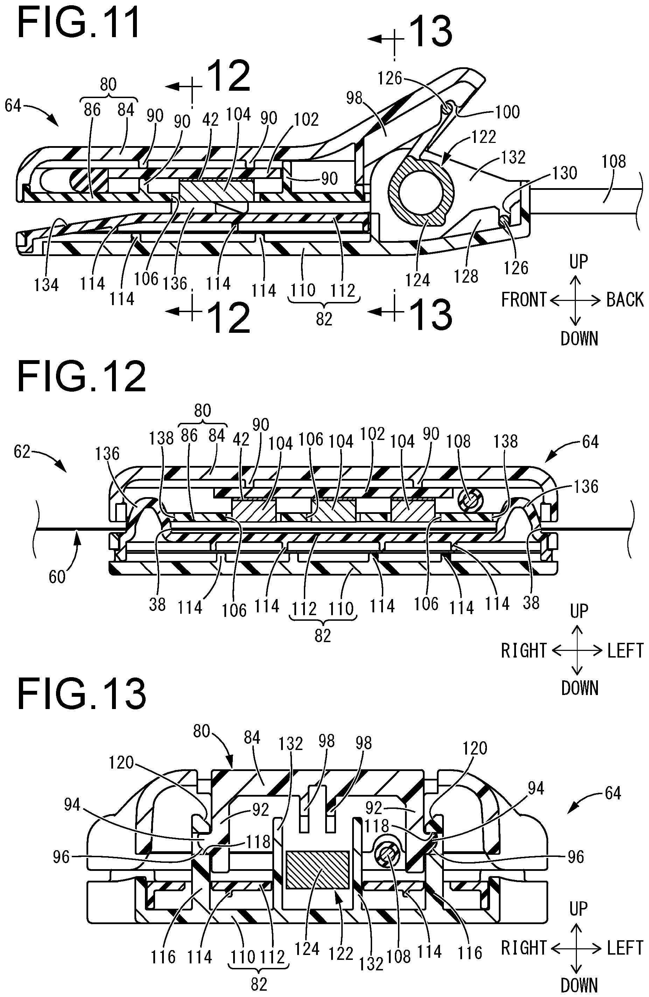

As shown in FIG. 13, spindle bearings 116 are provided at the portion of the lower outer member 110 extending backward from the lower inner member 112. The spindle bearings 116 are in the form of a pair of opposed plates protruding upward from the lower outer member 110, each of which is penetrated by a shaft insertion hole 118 in the direction of opposition. The protruding distal end faces of the pair of spindle bearings 116, 116 constitute second guide surfaces 120 that slope downward toward the inside in the direction of opposition.

A spring member 122 serving as a urging member is disposed between the opposed faces of the pair of spindle bearings 116, 116. The spring member 122 is a torsion coil spring including a tubular intermediate part 124 wound in a spiral shape, and terminal parts 126, 126 on opposite sides protruding peripherally outward from the tubular intermediate part 124. The terminal parts 126, 126 extend in mutually different directions that make an angle of approximately 90° in the circumferential direction of the tubular intermediate part 124 in the stationary state in which no external force is acting. Each end of the terminal parts 126, 126 is bent and extends in the left-right direction, which is the axial direction of the tubular intermediate part 124.

One terminal part 126 of the spring member 122 is attached to a second support rib 128. The second support rib 128 is integrally formed with the lower outer member 110, and projects upward from the lower outer member 110 behind the lower inner member 112. The second support rib 128 is penetrated by a second notch 130, which opens upward, in the thickness direction (the left-right direction). The end of the terminal part 126 of the spring member 122 is fitted into the second notch 130 and is rotatably supported.

A pair of positioning pieces 132, 132, which position the spring member 122 in the left-right direction, are integrally formed with the lower outer member 110. The positioning pieces 132, 132 project upward from the lower outer member 110. Regarding the spring member 122 attached to the lower outer member 110 at the terminal parts 126, the tubular intermediate part 124 is arranged between the opposed positioning pieces 132, 132, and the contact between the tubular intermediate part 124 and the positioning pieces 132, 132 limits the amount of displacement of the spring member 122 relative to the lower outer member 110 in the left-right direction. The second support rib 128, which supports the terminal part 126 of the spring member 122, is disposed between the opposed positioning pieces 132, 132 in the left-right direction.

As shown in FIG. 11, the upper surface of the front part of the lower inner member 112 comprises a sloping surface 134 that slopes downward toward the front. With this configuration, the lower jaw part 82 has a tapered shape whose thickness dimension in the vertical direction gradually decreases toward the front. The upper surface of the back part of the lower inner member 112 spreads in the direction approximately orthogonal to the vertical direction, so that the sloping surface 134 slopes relative to the upper surface of the bark part of the lower inner member 112.

As shown in FIG. 12, the left and right opposite end portions of the lower inner member 112 are provided with respective positioning pins 136, 136 protruding upward. The positioning pins 136 are integrally formed with the lower inner member 112, and are provided behind the sloping surface 134 (see FIG. 11). The positioning pin 136 of this practical embodiment is a hollow structure having a tapered outer shape whose diameter decreases toward the projecting distal end. The left and right opposite end portions of the upper inner member 86 are penetrated by pin insertion holes 138, 138 through which the positioning pins 136, 136 can be inserted.

As shown in FIG. 13, the upper jaw part 80 and the lower jaw part 82 are coupled to each other by insertion of the spindle parts 94, 94 into the shaft insertion holes 118, 118 of the spindle bearings 116, 116. The upper jaw part 80 and the lower jaw part 82 are attached to be mutually rotatable around the spindle parts 94, 94, and the front end of the upper jaw part 80 and the front end of the lower jaw part 82 are vertically displaceable relative to each other in the direction of mutual approach and in the direction of mutual separation.

In this practical embodiment, the spindle parts 94, 94 can be inserted into the shaft insertion holes 118, 118 by a simple operation of bringing the upper jaw part 80 closer to the lower jaw part 82 from above. That is, by bringing the upper jaw part 80 closer to the lower jaw part 82 from above, the first guide surface 96 of each spindle part 94 is pressed against the second guide surface 120 of each spindle bearing 116, and the spindle part 94 enters inside of the spindle bearing 116 in the left-right direction accompanied by elastic deformation of the shaft support part 92 and the spindle bearing 116. When the spindle part 94 reaches the shaft insertion hole 118, elastic deformation of both the shaft support part 92 and the spindle bearing 116 is released, so that the spindle part 94 is inserted into the shaft insertion hole 118.

The front ends of the upper jaw part 80 and the lower jaw part 82 are urged by the spring member 122 vertically in the direction of mutual approach. With one terminal part 126 of the spring member 122 being inserted in and rotatably supported by the second notch 130 of the second support rib 128 of the lower jaw part 82, the upper jaw part 80 is brought closer to be attached to the lower jaw part 82 from above. By so doing, the other terminal part 126 of the spring member 122 is inserted in and rotatably supported by the first notch 100 of the first support rib 98 of the upper jaw part 80. The lower surface of the first support rib 98 slopes upward toward the back, and due to downward displacement of the upper jaw part 80 toward the lower jaw part 82, the other terminal part 126 of the spring member 122 is guided along the lower surface of the first support rib 98 into the first notch 100. Thus, by means of assembly operation of bringing the upper jaw part 80 closer to the lower jaw part 82 in the vertical direction, the spring member 122 can be attached to the jaw parts 80, 82 . With the two terminal parts 126, 126 of the spring member 122 rotatably supported by the first notch 100 and the second notch 130, urging force based on elasticity of the spring member 122 acts between the upper jaw part 80 and the lower jaw part 82. Such urging force of the spring member 122 acts in the direction in which the back part of the upper jaw part 80 and the back part of the lower jaw part 82 are separated from each other in the vertical direction, and acts on the upper jaw part 80 and the lower jaw part 82 that are allowed to relatively rotate around the spindle part 94, in the direction in which the front parts of the upper jaw part 80 and the lower jaw part 82, which are located in front of the spindle part 94, approach each other. That is, in the connector 64, the front parts of the upper jaw part 80 and the lower jaw part 82 are held closed by the urging force of the spring member 122 when no external force is applied, and the front parts can be opened against the urging force of the spring member 122 by a downward input to the operating part 88.

The connector 64 is attached to the liquid detection sheet 60 (the base sheet 16) as shown in FIG. 6. That is, in the open state in which the front ends of the upper jaw part 80 and the lower jaw part 82 of the connector 64 are separated from each other by applying force to the operating part 88, the back end portion of the base sheet 16 is inserted between the upper jaw part 80 and the lower jaw part 82. In the open state of the upper jaw part 80 and the lower jaw part 82, the positioning pins 136, 136 are detached downward from the pin insertion holes 138, 138 of the upper jaw part 80, and the base sheet 16 can pass between the distal ends of the positioning pins 136, 136 and the lower surface of upper jaw part 80. The base sheet 16 is inserted deeper (backward) between the upper jaw part 80 and the lower jaw part 82 until the positioning holes 38, 38 reach a location corresponding to the positioning pins 136, 136.

Then, by releasing the input to the operating part 88, the positioning pins 136, 136 are inserted through the positioning holes 38, 38 of the base sheet 16 and the pin insertion holes 138, 138 of the upper jaw part 80, as shown in FIG. 12. By so doing, the base sheet 16 and the connector 64 are mutually positioned in the planar direction of the base sheet 16 by engagement of the positioning pins 136, 136 and the peripheries of the openings of the positioning holes 38,38. In addition, the distal end portions of the positioning pins 136, 136 that has passed through the positioning holes 38, 38 are inserted through the pin insertion holes 138, 138 of the upper jaw part 80, thereby preventing the positioning pins 136, 136 from unintentionally slipping out of the positioning holes 38, 38. In particular, in this practical embodiment, the upper jaw part 80 and the lower jaw part 82 are held in the closed state by being urged by the spring member 122. Thus, the inserted state of the positioning pins 136, 136 through the positioning holes 38, 38 is maintained with excellent reliability.

In the base sheet 16 inserted between the upper jaw part 80 and the lower jaw part 82 of the connector 64, the first and second terminals 30, 36, 36 of the first and second detection wirings 66, 68 are in contact with the internal terminals 104, 104, 104 of the connector 64, and the first and second detection wirings 66, 68 and the internal circuit 42 of the connector 64 are electrically connected. Therefore, the first and second detection wirings 66, 68 are electrically connected to the external wiring 108 via the internal circuit 42 of the connector 64.

In this manner, the connector 64 is positioned with respect to the base sheet 16 and is attached to the base sheet 16 with the internal circuit 42 of the connector 64 electrically connected to the first and second detection wirings 66, 68 of the base sheet 16.

The liquid detection sheet 60 including the connector 64 is laid on a bed for example, and the forearm 5 of the patient 1 is placed on the liquid detection sheet 60 as shown in FIG. 6. In this way, the liquid detection sheet 60 is used when detecting bleeding or leakage of liquid medicine from the puncture site of the indwelling needle (the blood collection port 3 and the blood return port 4) indwelled in the forearm 5 of the patient 1. The principle of detecting the detection target liquid (the blood, etc.) is the same as that in the first practical embodiment and is not described here.

The lower jaw part 82 of the connector 64 is configured such that the upper surface of the front part comprises the sloping surface 134 that slopes downward toward the front, which is the distal end attached to the base sheet 16, thereby becoming thinner in the vertical direction toward the front. This makes it easier to insert the lower jaw part 82 between the upper surface of the bed and the lower surface of the base sheet 16 when connecting the connector 64 to the base sheet 16 laid on the bed, thereby facilitating the connection operation of the connector 64.

The liquid detection sheet 60 of this practical embodiment cannot be turned upside down, since the base sheet 16 is liquid-impermeable, and the right side and the wrong side of the sheet are defined when in use. Therefore, in the liquid detection sheet 60 of this practical embodiment, a logo 140 that is asymmetrical on the right side and the wrong side is printed on the upper surface of the base sheet 16, and the right side and the wrong side of the liquid detection sheet 60 can be grasped by visually checking the said logo 140.

In FIG. 6, the liquid detection sheet 60 is illustrated for monitoring bleeding on the left arm of the patient 1, but the liquid detection sheet 60 can also be used for monitoring bleeding on the right arm of the patient 1. Since the base sheet 16 of the liquid detection sheet 60 is liquid-impermeable and cannot be used by being turned upside down, if laid under the right arm, the liquid detection sheet 60 is used in an orientation rotated 180° in the circumferential direction. Here, the first and second terminals 30, 36, 36 of the first and second detection wirings 66, 68 are arranged in the center portion in the left-right direction. Thus, even if the liquid detection sheet 60 is used in the orientation rotated 180° in the circumferential direction, those first and second terminals 30, 36, 36 are configured to be arranged in the center portion in the left-right direction. With this configuration, even when the orientation of the liquid detection sheet 60 differs by 180°, the change in position of the first and second terminals 30, 36, 36 (the connector 64) in the left-right direction is small. This makes it possible to avoid troubles such as an excess length of the external wiring 108 extending from the connector 64 becoming an obstacle, and the external wiring 108 being insufficient in length.

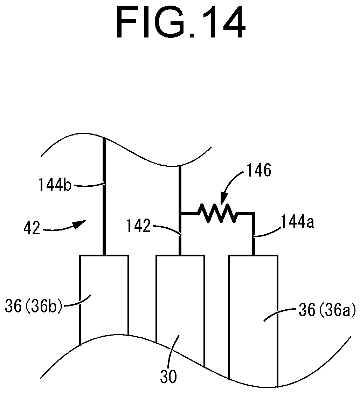

In this practical embodiment, in the internal circuit 42 of the connector 64 located outside the main detection region 37 of the liquid detection sheet 60, as shown in FIG. 14, a first wiring part 142 connected to the first terminal 30 of the first detection wiring 66 and a second wiring part 144a connected to one second terminal 36a of the second detection wiring 68 are connected by a resistor 146. The electrical resistance value of the resistor 146 is set larger than the electrical resistance value of the portion short-circuited by the detection target liquid in the liquid detection sheet 60 (the electrical resistance value of the liquid-permeable sheet 18 that has absorbed the detection target liquid between the first and second detection wirings 66, 68). By applying a voltage between the first wiring part 142 connected to the first terminal 30 and a second wiring part 144b connected to the other second terminal 36b, the connection state between the connector 64 and the base sheet 16, as well as presence or absence of disconnection in the second detection wiring 68 are checked, and the detection target liquid is detected.

The detection circuit can be checked by applying a voltage between the first wiring part 142 and the second wiring part 144b in the state where the first and second detection wirings 66, 68 are not short-circuited by the detection target liquid. Specifically, if the connector 64 and the base sheet 16 are not properly connected or if the second detection wiring 68 is disconnected, no current flows, so that the detection current value of the detector 52 becomes zero. Meanwhile, if the connector 64 and the base sheet 16 are properly connected and the second detection wiring 68 is not disconnected, the current flows through a circuit with a high resistance value, including the resistor 146, so that the detector 52 detects a low current value. Therefore, the presence or absence and the magnitude of the detected current can be used to check the connection state between the connector 64 and the base sheet 16 , as well as the presence or absence of disconnection in the second detection wiring 68. As in the first practical embodiment, since the first detection wiring 66 is surrounded by the second detection wiring 68, it is unlikely that only the first detection wiring 66 is damaged. Thus, by checking whether the second detection wiring 68 is damaged, it is also possible to check whether the first detection wiring 66 is damaged.

If the first and second detection wirings 66, 68 are short-circuited by the detection target liquid, the current flows through a circuit with a law resistance value that does not include the resistor 146, so that the detector 52 detects a higher current value than in the case where there is no short circuit. Therefore, the presence or absence of the detection target liquid in the main detection region 37 of the liquid detection sheet 60 can be determined by the magnitude of the detected current.

Thus, the liquid detection device 62 of this practical embodiment does not need to switch the energization state from the connector 64 to the first and second detection wirings 66, 68 when checking the detection circuit and when monitoring the detection target liquid. In addition, the liquid detection device 62 of this practical embodiment can simultaneously check the detection circuit and monitor the detection target liquid.

The resistor 146 is provided at a position outside the main detection region 37 of the liquid detection sheet 60, and in this practical embodiment, the resistor 146 is provided to the internal circuit 42 of the connector 64 that is connected to the base sheet 16. This allows a greater degree of freedom in selecting the resistor 146 than when the resistor is provided in the main detection region 37 of the liquid detection sheet 60. In addition, by arranging the resistor 146 at a position outside the main detection region 37 with which the detection target liquid comes into contact, it is possible to prevent the detection target liquid from sticking to the resistor 146. Besides, while the external wiring 44 of the first practical embodiment includes three core wires comprising conductors that are connected to the first and second terminals 30, 36, 36, the external wiring 108 of this practical embodiment includes two core wires that are connected to the first and second terminals 30, 36, so that the external wiring 108 can be made thinner.

In the liquid detection sheet 60, the distance between the first detection wiring 66 and the second detection wiring 68 is set within the range of 40 to 100 mm. This makes it possible to reduce the frequency of occurrence of false detection caused by perspiration or the like. In addition, detection target liquids that accumulate on the liquid-impermeable base sheet 16 in excess of a predetermined amount, such as blood leakage and liquid medicine leakage can be effectively detected by the short circuit between the first and second detection wirings 66, 68.

In the liquid detection sheet 60 of this practical embodiment, the distance between the first detection wiring 66 and the second detection wiring 68 is made approximately constant in the main detection region 37, in which the detection target liquid can be detected, by means of the first curved part 70 and the corner-shaped part 74 of the first detection wiring 66, and the second curved parts 72a, 72b of the second detection wiring 68. Therefore, regardless of the sticking position of the detection target liquid in the main detection region 37 of the liquid detection sheet 60, the lower limit of the detectable liquid amount is stabilized, thereby making it possible to detect the predetermined amount of the detection target liquid in a more reliable manner.

The liquid detection sheet 60 can detect the detection target liquid with excellent reliability because the base sheet 16 has liquid-impermeable properties that prevent the passage of the detection target liquid so that the detection target liquid accumulates on the base sheet 16. Besides, it is difficult for the detection target liquid to pass through the base sheet 16 and leak downward. Thus, when the liquid detection sheet 60 is laid on the bed, for example, contamination of the bed due to sticking of the detection target liquid will be avoided.

The liquid detection sheet 60 has a three-layer structure with a small number of layers, in which the first and second detection wirings 66, 68 are formed by printing on the base sheet 16, thereby facilitating manufacture, reducing costs, and the like. The liquid-permeable sheet 18 covering the upper surface of the base sheet 16, on which the first and second detection wirings 66, 68 are formed by printing, is made of nonwoven fabric. Thus, a good tactile feeling can be provided to the upper surface of the liquid detection sheet 60, which is to be in contact with the forearm 5 of the patient 1. Besides, the detection target liquid is absorbed by the nonwoven fabric and spreads out, thereby inhibiting the detection target liquid from spilling or sticking to the patient 1. In addition, by heating the adhesive sheet 20 with the base sheet 16, the adhesive sheet 20, and the liquid-permeable sheet 18 laminated, the base sheet 16 and the liquid-permeable sheet 18 can be adhered to each other utilizing, for example, the heat-welding action of thermoplastic resin or the like, without requiring an adhesive application process.

While the present invention has been described in detail hereinabove in terms of the practical embodiments, the invention is not limited by the specific description thereof. For example, the wiring patterns of the first and second detection wirings 22, 24 are merely exemplary and are not limited in particular. However, in consideration of preventing false detection and reliably obtaining an effective detection area that enables stable detection, it is desirable that the remote distance between the first and second detection wirings 22, 24 be as constant as possible. For example, in order to prevent the remote distance between the first and second detection wirings 22, 24 from becoming partially larger at the corners, the corners may be rounded in an arcuate shape or the like, as shown in the second practical embodiment.

The preceding first and second practical embodiments describe a three-terminal type wiring structure in which one first terminal 30 and two second terminals 36, 36 are provided, but four or more terminals may be provided. Specifically, for example, a four-terminal type wiring structure with two first terminals 30, 30 and two second terminals 36, 36 may be adopted. This structure enables checking for disconnection not only in the second detection wiring 24 but also in the first detection wiring 22 by applying a check voltage. There may be three or more detection wirings, and a detection voltage may be applied between each of those three or more detection wirings to enable detection of liquid.

In the preceding first and second practical embodiments, the base sheet 16 protrudes backward from the liquid-permeable sheet 18 and the adhesive sheet 20, and the positioning holes 38 are provided only to the base sheet 16 in the said protruding portion. However, for example, the positioning holes 38 can be provided so as to penetrate not only the base sheet 16 but also the liquid-permeable sheet 18 and the adhesive sheet 20. The base sheet 16, the liquid-permeable sheet 18, and the adhesive sheet 20 may have the same size as one another, or alternatively, for example, the liquid-permeable sheet 18 may be larger than the base sheet 16 and protrude radially outward from the base sheet 16. The connector attached to the base sheet 16 may be part of the liquid detection sheet 10, or may be a separate component from the liquid detection sheet 10 to constitute the liquid detection device.

In the preceding first and second practical embodiments, the reinforcing part 40 is provided at the periphery of the opening of the positioning hole 38, but the reinforcing part 40 is not essential. Besides, the reinforcing part 40 does not necessarily have to protrude from the base sheet 16 outward in the thickness direction. For example, the reinforcing part 40 can be embedded in the periphery of the opening of the positioning hole 38 in the base sheet 16 so as not to protrude from the base sheet 16 outward in the thickness direction.

The positioning part is not necessarily limited to the one constituted by a through hole such as the positioning hole 38. For example, it is also possible to position the base sheet 16 and the connector 14 by forming a recess opening downward in the base sheet 16 and inserting a convex part protruding from the lower jaw part 46 of the connector 14 into the said recess, in which case the positioning part is constituted by the said recess. It is also possible to provide a positioning pin or a convex part on the base sheet 16 side and to provide a positioning hole or a recess on the connector 14 side, so that the positioning part is constituted by the positioning pin or the convex part.

The preceding first and second practical embodiments show a thin-walled base sheet 16 as an example of the base member. However, the base member is not limited to a thin-walled flexible member such as a sheet or a film, but may be in the form of a plate or the like, for example. The base sheet 16 may be made of a liquid-permeable material that allows passage of the detection target liquid. In this case, for example, a liquid-impermeable sheet as a separate element from the base sheet 16 may be laminated below the base sheet 16 so that the passage of the detection target liquid is prevented below the detection wiring. Thus, the liquid-impermeable member is not necessarily limited to the one constituted by the base sheet 16.

The preceding first and second practical embodiments show an example in which the forearm 5 of the patient 1 is placed on the liquid detection sheet 10 so as to extend in the direction approximately parallel to the branched wiring parts 26b, 26c, 32b, 32c, 34 of the first and second detection wirings 22, 24 (in the left-right direction). However, the liquid detection device 12 can also be used with the forearm 5 placed on the liquid detection sheet 10 so as to extend in the direction intersecting the branched wiring parts 26b, 26c, 32b, 32c, 34 of the first and second detection wirings 22, 24 (for example, in the front-back direction).

The detector 52 shown in FIG. 5 is separate from the artificial dialysis device 2 and is attached to the artificial dialysis device 2 by, for example, being hung by a hook on the artificial dialysis device 2. However, the detector 52 can also be built into the artificial dialysis device 2.

The preceding first and second practical embodiments show an example of using the liquid detection device 12 including the liquid detection sheet 10 for medical use to detect blood leakage during artificial dialysis. However, the liquid detection sheet 10 can also be used to detect liquid leakage during, for example, infusion or medication. The detection target liquid to be detected using the liquid detection sheet 10 for medical use is not limited to blood, but may also be, for example, biological fluids in the human body (biological fluids in a broad sense including liquids secreted in the body (such as saliva) and liquids excreted outside the body (such as urine)), medical liquids such as liquid medicine and infusion liquid, and the like.

The preceding first and second practical embodiments show an example in which the blood 6 is detected when the blood 6 touches both the first detection wiring 22 (66) and the second detection wiring 24 (68), causing those first detection wiring 22 (66) and second detection wiring 24 (68) to be mutually conducted. However, it is also possible, for example, to make a detection target liquid detectable with the detection target liquid such as blood being remote from at least one of the first detection wiring 22 (66) and the second detection wiring 24 (68). Specifically, for example, the presence of the detection target liquid may be detected based on a change in the electrical connection state between the first detection wiring 22 (66) and the second detection wiring 24 (68) (for example, a change in electrical resistance value) due to the detection target liquid approaching the detection wiring in a state of separation.

### KEYS TO SYMBOLS

10 liquid detection sheet (first practical embodiment)
12 liquid detection device
14 connector
16 base sheet (base member)
18 liquid-permeable sheet (nonwoven fabric)
20 adhesive sheet (heat-welding sheet)
22 first detection wiring
24 second detection wiring
26 first central detection part
26a right end (opposite wiring part)
26b front end (branched wiring part)
26c back end (branched wiring part)
28 terminal connecting part
30 first terminal
32 outer circumferential detection part
32a left end (opposite wiring part)
32b front end (branched wiring part)
32c back end (branched wiring part)
32d right end
34 second central detection part (branched wiring part)
36 second terminal
36a second terminal
36b second terminal
37 main detection region
38 positioning hole (positioning part)
40 reinforcing part
42 internal circuit
44 external wiring
46 lower jaw part
48 upper jaw part
50 positioning pin
52 detector
54 state checking member
60 liquid detection sheet (second practical embodiment)
62 liquid detection device
64 connector
66 first detection wiring
68 second detection wiring
70 first curved part (curved part)
72a second curved part (curved part)
72b second curved part
74 corner-shaped part
76 line
78 marker
80 upper jaw part
82 lower jaw part
84 upper outer member
86 upper inner member
88 operating part
90 reinforcing rib
92 shaft support part
94 spindle part
96 first guide surface
98 first support rib
100 first notch
102 internal substrate
104 internal terminal
106 through hole
108 external wiring
110 lower outer member
112 lower inner member
114 reinforcing rib
116 spindle bearing
118 shaft insertion hole
120 second guide surface
122 spring member (urging member)
124 tubular intermediate part
126 terminal part
128 second support rib
130 second notch
132 positioning piece
134 sloping surface
136 positioning pin
138 pin insertion hole
140 logo
142 first wiring part
144a second wiring part
144b second wiring part
146 resistor
1 patient
2 artificial dialysis device
3 blood collection port
4 blood return port
5 forearm
6 blood (detection target liquid)

## Claims

1. A liquid detection sheet for medical use comprising:
a base member having electric insulation properties; and
a first detection wiring and a second detection wiring provided remote from each other on the base member, the liquid detection sheet being configured to detect leakage of a detection target liquid such as blood based on a change in an electrical connection state between the first detection wiring and the second detection wiring due to sticking of the detection target liquid, wherein
a main detection region in which the first detection wiring and the second detection wiring extend substantially parallel to each other is provided on the base member, and
a distance between the first detection wiring and the second detection wiring opposed to each other in the main detection region is set within a range of 40 to 100 mm.

2. A liquid detection sheet for medical use comprising:
a base member having electric insulation properties; and
a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein
an upper surface of the base member on which the plurality of detection wirings are formed by printing is covered with nonwoven fabric, and
the base member and the nonwoven fabric are adhered to each other by a heat-welding sheet.

3. A liquid detection sheet for medical use comprising:
a base member having electric insulation properties; and
a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein
a liquid-impermeable member that prevents passage of the detection target liquid is arranged below the plurality of detection wirings.

4. The liquid detection sheet according to claim 3, wherein terminals of the plurality of detection wirings are located at a widthwise center portion of the base member in an outer peripheral end of the base member.

5. A liquid detection sheet for medical use comprising:
a base member having electric insulation properties; and
a first detection wiring and a second detection wiring provided remote from each other on the base member, the liquid detection sheet being configured to detect leakage of a detection target liquid such as blood based on a change in an electrical connection state between the first detection wiring and the second detection wiring due to sticking of the detection target liquid, wherein
the second detection wiring is provided so as to surround the first detection wiring on the base member.

6. The liquid detection sheet according to claim 5, wherein
the first detection wiring and the second detection wiring are each imparted with a comb teeth-like shape by opposite wiring parts extending substantially parallel to each other and a plurality of branched wiring parts extending from the respective opposite wiring parts in a direction of opposition of the opposite wiring parts, and
the plurality of branched wiring parts of the first detection wiring and the plurality of branched wiring parts of the second detection wiring are arranged in a length direction of the opposite wiring parts so as to interdigitate with one another in the direction of opposition of the opposite wiring parts.

7. The liquid detection sheet according to claim 5 or 6, wherein
a first terminal is provided at one end portion of the first detection wiring,
a pair of second terminals are provided at two end portions of the second detection wiring, and
the first terminal and the pair of second terminals are arranged side by side in a straight line, and the first terminal is located between the pair of second terminals.

8. The liquid detection sheet according to any one of claims 5-7, wherein
the base member comprises a base sheet that is impermeable to the detection target liquid,
the first detection wiring and the second detection wiring are formed by printing on an upper surface of the base sheet, and
a liquid-permeable sheet that has electric insulation properties and allows passage of the detection target liquid is provided on the upper surface of the base sheet so as to cover the first detection wiring and the second detection wiring.

9. The liquid detection sheet according to any one of claims 5-8, further comprising a state checking member connected to the two end portions of the second detection wiring and configured to check an electrical state of the second detection wiring by applying a check voltage to the second detection wiring.

10. A liquid detection sheet for medical use comprising:
a base member having electric insulation properties; and
a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein
the plurality of detection wirings are connected to each other by a resistor at a position outside a main detection region configured to detect the leakage of the detection target liquid.

11. A liquid detection sheet for medical use comprising:
a base member having electric insulation properties; and
a first detection wiring and a second detection wiring provided remote from each other on the base member, the liquid detection sheet being configured to detect leakage of a detection target liquid such as blood based on a change in an electrical connection state between the first detection wiring and the second detection wiring due to sticking of the detection target liquid, wherein
one of the first detection wiring and the second detection wiring includes a curved part spaced outward from an end of the other of the first detection wiring and the second detection wiring and extending in an arc-like shape.

12. A liquid detection sheet for medical use comprising:
a base member having electric insulation properties; and
a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein
terminals of the plurality of detection wirings are arranged side by side in an outer peripheral portion of the base member, and
positioning parts are provided to the base member on opposite sides in a direction of arrangement of the terminals and located close to the terminals, the positioning parts positioning a connector with respect to the base member, the connector including an external wiring electrically connected to the terminals.

13. The liquid detection sheet according to claim 12, wherein
the base member comprises a base sheet that is impermeable to the detection target liquid, and
the base sheet is provided with a positioning hole through which a positioning pin protruding from the connector is inserted, and the positioning parts include the positioning hole.

14. The liquid detection sheet according to claim 13, wherein an annular reinforcing part is provided at a periphery of an opening of the positioning hole of the base sheet, the annular reinforcing part protruding in a thickness direction of the base sheet.

15. A liquid detection sheet for medical use comprising:
a base member having electric insulation properties; and
a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein
a connector including an internal circuit electrically connected to terminals of the plurality of detection wirings is provided, and
an external wiring electrically connected to the internal circuit of the connector and extending to an outside from the connector is configured such that the external wiring extends from the internal circuit to a side on which the connector is connected to the terminals, and an extending end of the external wiring curves so as to make a U-turn and extends to the outside from the connector on a side opposite to the side on which the connector is connected to the terminals.

16. A liquid detection sheet for medical use comprising:
a base member having electric insulation properties; and
a plurality of detection wirings provided remote from each other on the base member, the liquid detection sheet being configured to detect leakage of a detection target liquid such as blood based on a change in an electrical connection state between the plurality of detection wirings due to sticking of the detection target liquid, wherein
a connector including an internal circuit electrically connected to terminals of the plurality of detection wirings is provided,
a distal end portion of the connector includes an upper jaw part and a lower jaw part that are relatively displaceable in a direction of mutual separation and in a direction of mutual approach,
an external wiring is electrically connected to the terminals via the internal circuit of the connector by an outer peripheral portion of the base member being clasped between the upper jaw part and the lower jaw part in a vertical direction, and
the lower jaw part of the connector arranged below the base member is configured such that an upper surface comprises a sloping surface that slopes downward toward a distal end of the connector attached to the base member whereby the lower jaw part becomes thinner in the vertical direction toward the distal end of the connector attached to the base member.

17. The liquid detection sheet according to claim 16, wherein the connector includes an urging member that urges distal end portions of the upper jaw part and the lower jaw part attached to the base member in the direction of mutual approach.
